(19) Europäisches Patentamt
European Patent Office
Office européen des brevets

(11) Publication number : **0 533 492 A2**

(12) **EUROPEAN PATENT APPLICATION**

(21) Application number : **92308534.4**

(22) Date of filing : **18.09.92**

(51) Int. Cl.$^5$ : **A61K 39/29,** C12P 21/02, C12N 1/19, // C12N15/51

(30) Priority : **20.09.91 US 762678**

(43) Date of publication of application : **24.03.93 Bulletin 93/12**

(84) Designated Contracting States : **AT BE CH DE DK ES FR GB GR IE IT LI LU MC NL PT SE**

(71) Applicant : **AMGEN INC.**
**Amgen Center, 1840 Dehavilland Drive**
**Thousand Oaks, CA 91320-1789 (US)**

(72) Inventor : **Langley, Keith E.**
**714 Danvers Circle**
**Newbury Park, California 93012 (US)**
Inventor : **Sachdev, Raj Kumar**
**5952 Palomar Circle**
**Camarillo California 93012 (US)**
Inventor : **Bitter, Grant A.**
**29126 Old Mill Creek Lane**
**Agoura, California 91301 (US)**
Inventor : **Fieschko, John C.**
**29131 Garden Oaks Court**
**Agoura Hills, California 91301 (US)**

(74) Representative : **Meddle, Alan Leonard et al**
**FORRESTER & BOEHMERT**
**Franz-Joseph-Strasse 38**
**W-8000 München 40 (DE)**

(54) **A hepatitis B vaccine formulation incorporating a bile acid salt.**

(57) A novel, highly immunogenic hepatitis B vaccine formulation comprising HBsAg produced as the result of recombinant yeast host cell expression and a bile salt, particularly sodium deoxycholate, are disclosed, as are the methods used to produce the vaccine. These methods include novel yeast culture conditions and novel HBsAg purification and adjuvanting protocols. In addition, methods utilizing these novel HBsAg formulations to immunize mammals, particularly humans, susceptible to hepatitis B infection are also described.

FIGURE 2

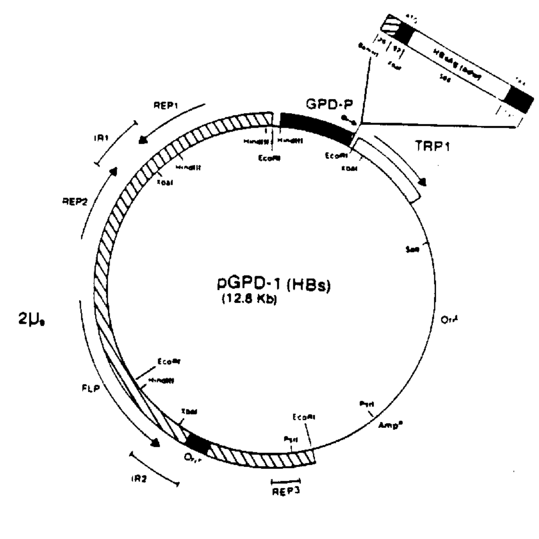


EP 0 533 492 A2

## FIELD OF THE INVENTION

The present invention relates to hepatitis vaccines and their formulation. Generally, this invention concerns the production of a Hepatitis B antigen in a microbial expression system and the subsequent incorporation of the purified immunogenic molecule into a formulation containing a secondary bile salt. Particularly, the present invention teaches the low temperature expression in yeast of the Hepatitis B Surface Antigen molecule, which is then purified and included in a vaccine formulation containing sodium deoxycholate.

## BACKGROUND OF THE INVENTION

The Hepatitis B virus (HBV) causes the disease now known as Hepatitis B, which formerly had been called "serum hepatitis". Worldwide, it is estimated that more than 200 million people are persistently infected with HBV. The Centers for Disease Control estimate that there are approximately 0.7 to 1.0 million chronic carriers of Hepatitis B in the United States and that this number is increasing 2 - 3% (8,000 - 16,000 individuals) annually. Hepatitis B viral infection is known to be a major cause of acute liver disease. Carriers of the Hepatitis B virus are at a high risk of contracting liver cirrhosis, massive hepatic necrosis, chronic acute hepatitis, and hepatocellular carcinoma. Blood and blood products are the predominant vehicles for HBV transmission, although viral antigens have also been found in tears, breast milk, saliva, urine, semen, and vaginal secretions. In addition, HBV is quite stable and is capable of surviving for days on environmental surfaces exposed to contaminated bodily fluids. Infection may occur when HBV, transmitted by infected bodily fluids, penetrates mucosal surfaces or enters the body through accidental or intentional breaks in the skin. The United States Public Health Service Immunization Practices Advisory Committee (IPAC) currently recommends pre-exposure prophylaxis with a Hepatitis B vaccine for individuals at high risk of exposure to Hepatitis B infection or virus (e.g. health care professionals, sexual partners of infected individuals). In addition, post-exposure prophylaxis employing active immunization with a Hepatitis B vaccine and passive immunization with Hepatitis B immune globulin (HBIg) is recommended for neonates born to women who test positive for Hepatitis B surface antigen (HBsAg) and for individuals exposed to HBV or HBsAg-positive material [*American Hospital Formulary Service,* (1990), pub. American Society of Hospital Pharmacists, pp. 1931-1938].

The human Hepatitis B virus has been associated with the Dane particle. This particle is found in the blood serum of carriers and has been identified as the causative agent in clinical Hepatitis B infection [WIPO Int. Pub. No. WO 87/01129; U.S. Patent No. 4,959,323, hereby incorporated by reference]. The Dane particle is a spherically shaped membrane structure having a 42 nanometer (nm) diameter. The particle is composed of lipids, DNA, and at least 4 antigenically distinct surface and core protein components. HBsAg, Hepatitis B Core Antigen (HBcAg), Hepatitis B e Antigen (HBeAg, a core-associated antigenic complex correlating with high infectivity), and a DNA polymerase [*American Hospital Formulary Service,* (1990), Eds. American Society of Hospital Pharmacists]. Also found in the blood serum of carriers is a 22 nm diameter sphere comprised of lipid particles containing HBsAg, but none of the other Dane particle components [WO 87/01129, supra]. Hepatitis B vaccines currently in use around the world utilize the 22 nm lipid HBsAg-containing particle isolated from either the plasma of human Hepatitis B carriers, from the culture of Hepatitis B-infected malignant mammalian cell lines, or from microorganisms genetically engineered to express the HBsAg gene, to prime the immune system of vaccinated individuals for resistance to future Hepatitis B infection. Presently, the United States Food and Drug Administration has granted approval for human use in this country of several Hepatitis B vaccines, both plasma-derived and recombinant. In addition, several other Hepatitis B vaccines, not approved by the FDA for use in the U.S., are being employed in other countries of the world. The Hepatitis B vaccines currently available worldwide confer resistance to infection from HBV and all its known serotypes (HBVa; adw, adr; ayr, ayw) upon those individuals who have been vaccinated. Additionally, Hepatitis B vaccination should prevent Hepatitis D, caused by the delta virus, because the delta virus replicates only in the presence of HBV infection. None of these vaccines, however, prevent Hepatitis caused by other agents, such as Hepatitis A virus, non-A, non-B Hepatitis viruses, or other viruses known to infect the liver.

Those vaccines presently available in the United States require three vaccinations to confer HBV immunity over an extended period. Typically, the second immunization is given one month after the first, and the third and final injection is administered six months after the initial dose [*American Hospital Formulary Service,* supra]. Responsiveness to HBV vaccines is dependent upon the age of the patient being immunized. The seroconversion rate (a measurement indicating the development of antibodies in response to infection or vaccination) of HBsAg in children under 10 years of age approaches 100%. For adults 20-39, seroconversion rates range from 95-99%, while adults 40 and older have seroconversion rates of about 91% [*Physicians' Desk Reference,* (1990), 44th ed., pub. E. Barnhart, pp:1441-1443].

Human plasma used in the production of Hepatitis B vaccines can be isolated from the blood of HBV infected individuals who are asymptomatic and have elevated HBV titers. In such carriers, the HBsAg concentration typically found is about 400 micrograms per milliliter (μg/mL) of whole blood. Because the total protein concentration of human plasma is about 60 milligrams per liter (mg/l), only 150-fold purification of the 22 nm particle is required [Chanock et al. (1984) eds. Cold Spring Harbor Laboratory, Cold Spring Harbor, N.Y., pp. 251-256]. However, because this type of vaccine (often called a first generation vaccine) is derived from human plasma, the supply is limited. In addition, the theoretical possibility that other contaminating infectious blood-borne viruses, such as the human immuno-deficiency virus (HIV), may be present in the serum raises significant concerns as to whether such serum-derived vaccines are safe. However, despite these concerns, one such plasma-derived Hepatitis B vaccine, manufactured by Merck Sharp & Dohme, was sold in the U.S. under the trade name Heptavax-B®. However, Heptavax-B® has voluntarily been withdrawn from the U.S. market by Merck and is thus no longer available in this country.

The Heptavax-B® production process employs purification and inactivation steps to remove infectious Hepatitis B viral particles in addition to known groups of animal viruses, including rhabdo virus, pox virus, toga virus, reo virus, herpes virus, corona virus, myxo virus, picorna virus, parvo virus, and retrovirus, delta agent, and slow viruses. Initial purification is accomplished by a double ultracentrifugation procedure, followed by three sequential inactivation procedures. pepsin cleavage at pH 2, 8M urea treatment, and finally the addition of formalin to 0.01%. Once purified and inactivated, the HBsAg preparation is formulated into a suspension suitable for intramuscular injection. All currently available Hepatitis B vaccines are administered intramuscularly, although subcutaneous administration is acceptable for those patients at risk for hemorrhage following intramuscular injection. HBsAg is formulated into a simulated physiological solution containing not more than 0.62 mg/mL of alum adjuvant (aluminium hydroxide). Additionally, thimersol is added as a preservative. For a review of serum-derived HBsAg purification techniques, see Field et al., (1988) *J. Virol. Methods,* vol 22. 283-94.

Another approach to obtaining Hepatitis B vaccines involves the infection of a cultured malignant mammalian cell line, specifically hepatoma cells, with the Hepatitis B virus. HBsAg may then be harvested from lysed preparations of these cells [Knowles et al., PCT Application No. PCT/US81/00778]. Although this approach eliminates the need for human plasma, and thus the possible presence of contaminating infectious agents, the undesirability of using neoplastic cell products in vaccines, the need for extreme caution to avoid infectivity, and the difficulties inherent in mammalian cell culture all serve to limit the practicality of this approach.

Alternatively, recombinant DNA technology enables the construction of expression vehicles capable of directing the expression of exogenous protein products in numerous host microorganisms, both eucaryotic and procaryotic. Often used eucaryotic host cells include yeast, such as *Saccharomyces cerevisiae,* and mammalian cell lines such as Chinese hamster ovary (CHO) cells. Examples of procaryotic organisms commonly used to direct the expression of foreign proteins include *Escherichia coli* and *Bacillus subtilus.*

Monkey kidney cells transfected with recombinant plasmids containing the HBsAg gene can release HBsAg either by cell lysis or by secretion [Levinson et al., EPO Application No. 73,656]. In addition, high level secretion of HBsAg can be achieved by fusing rat or monkey primary hepatocytes with transfected mouse or monkey cell lines expressing the antigen at low levels [Streeck et al., (1988) *Technological Advances in Vaccine Production,* Ed. Alan R. Liss, Inc., pp. 157-166]. Use of such cell lines largely eliminates the concerns related to the use of cell products from cancerous cells in addition to lessening the likelihood of contaminating infectious agents occurring in the vaccine preparation. However, vaccines derived as the expression products of mammalian cell lines (either chimeric or non-fused) may give rise to concerns about the presence of small quantities of undesirable residual mammalian proteins in the vaccine formulation, which may potentially lead to unfavorable immunogenic reactions in HBsAg-immunized subjects. Thus, Hepatitis B vaccines free from association with any mammalian proteins are preferred.

One approach enabling the production of a HBsAg-containing Hepatitis B vaccine free from association with any mammalian protein is to engineer a procaryotic organism, like *E. coli*, to produce the antigenic molecule. However, such attempts have been shown to lead to low polypeptide yields due to the degradation of HBsAg in bacteria [Rutter et al., European Patent Application No. 020,251] and because HBsAg inhibits the growth of the bacterial microorganism [Miyanhara et al., European Patent Application No. 105,049]. Further, the protein component of HBsAg normally undergoes substantial post-translational modification when produced by infected mammalian cells. Typical mammalian post-translational modification includes glycosylation at specific polypeptide sequences and possible phosphorylation and/or methylation. In human plasma, approximately 70-80% of the HBsAg produced in infected individuals is glycosylated. The cellular machinery responsible for such post-translational modification in mammalian cells is not present in procaryotic organisms. Thus, *E. coli* is unable to covalently link, as well as produce, the carbohydrate moieties normally associated with the protein component of HBsAg.

For these reasons, non-mammalian eucaryotic microorganisms or cell lines capable of expressing heterologous proteins are preferred for use in the production of HBsAg suitable for vaccine incorporation. Such transformable eucaryotic cell lines include various yeasts, insect cells, etc. When produced in such recombinant eucaryotic microorganisms, the HBsAg protein may undergo the post-translational modifications mentioned above. The HBsAg so produced may generate the desired immune system priming to HBV in vaccinated individuals. The HBsAg protein used in all current recombinant Hepatitis B vaccines is produced in yeast and is derived from the HBV serotype adw [*American Hospital Formulary Service,* supra, pp. 1931-1938]. Yeast is the preferred organism because it has no known pathogenic relationship with man, lacks endotoxins and lytic viruses, and is a GRAS (generally recognized as safe) organism.

The surface proteins of HBV, which provide the epitopes most likely to be recognized and identified as those of an invading foreign substance by the immune system of the infected individual, are known to be the translational products of the HBV large open reading frame (ORF) containing three separate domains, preS1, preS2 and S. Each ORF is in frame with the next and each begins with an ATG translation initiation codon. Thus, these three domains define three polypeptides, S (comprised of 226 amino acids and representing the entire HBsAg molecule), preS2 + S (281 amino acids), and preS1 + preS2 + S (386 amino acids). Genetic engineering has enabled the construction of various microbial expression systems coding solely for the production of HBsAg, as is done in the instant invention.

The Recombivax HB® vaccine (Merck Sharp & Dohme) was approved for human use in 1986 and incorporates the 226 amino acid HBsAg molecule into a suitable vaccine formulation. The 226 amino acid S protein has an apparent molecular weight of 24,000 daltons (24 kD). The HBsAg protein utilized in this vaccine is the product of recombinant yeast expression. The protein monomers aggregate to form 20 nm spherical particles, slightly smaller than the 22 nm HBsAg-containing particle isolated from the serum of infected individuals. Bitter et al. [(1988) *J. Med. Virol.*, vol. 25, pp:123-140] suggest that the HBsAg particles formed as a result of yeast expression may largely be assembled during cell lysis procedures. Extensive immunological comparisons between plasma-derived HBsAg and HBsAg derived from recombinant yeast expression show the proteins to be similar in eliciting immunogenic responses in both *in vitro* and *in vivo* biological assays. Further, the preS2 + S polypeptide, which has a molecular weight of 34 kD, also assembles into 22 nm particles and is equal immunogenically to the HBsAg protein in mice and monkeys [Ellis et al., (1988) *Technological Advances in Vaccine Development,* Ed. Arthur R. Liss, Inc., pp. 127-136]. The preS1 + preS2 + S protein does not aggregate to form 22 nm spherical particles and is less immunogenic than either preS2 + S or HBsAg vaccine preparations. SmithKline has also produced a yeast-derived Hepatitis B vaccine comprised of HBsAg, called Engerix-B®, which consists of the same polypeptide product as that used in Recombivax HB® [*American Hospital Formulary Service,* supra, pp. 1932]. Engerix-B® has also been approved for human use in the United States.

The recombinant HBsAg particles utilized in the Recombivax HB® and Engerix-B® vaccines are produced in the yeast *S. cerevisiae.* In each of these vaccines, intact HBsAg-containing particles are first purified and then treated with formaldehyde prior to absorption onto alum. Because the HBsAg isolated in these procedures is the product of non-mammalian host cell expression, protocols for use in the production of plasma-derived Hepatitis B vaccines designed to inactivate contaminating viruses capable of infecting the vaccinated patient are unnecessary. Once HBsAg is purified and absorbed onto alum in a buffer suitable for human injection, thimersol is added.

Current methods for the production of recombinant products in yeast, including HBsAg, involve cultivation of the microorganism at temperatures of about 25 - 30°C. Yeast can be grown in either a batch or continuous growth format. Typically, the culture is grown under glucose limitation to minimize the formation of ethanol, a known yeast growth inhibitor. Should glucose concentrations rise above 0.2 - 0.5%, partial repression of oxidative metabolism and the production of ethanol occurs, even under aerobic conditions. Glucose limited growth allows for the production of higher cell densities and therefore enables the production of greater quantities of recombinant derived products per unit volume. See Fieschko et al. [(1987) *Biotechnology and Bioengineering,* vol. 29, pp. 1113-1121] for a description of a media formulation enabling the production of grams of recombinant product per liter in *S. cerevisiae* cultured under carbon limited conditions. In such a method, glucose, one carbon source utilized by yeast to generate cellular energy (adenosine triphosphate, ATP), is added to the culture medium at a rate below that of the specific growth rate possible for the yeast at a given temperature. Addition of glucose at this rate necessarily slows the specific growth rate of the cultivated yeast, thus insuring that the much more efficient process of oxidative metabolism, rather than glycolysis, is employed to generate ATP. However, ethanol will also be produced if the specific growth rate of yeast rises above 0.2-0.25$h^{-1}$. Woehrer et al. [(1981) *Biotechnol. Bioeng.*, vol. 23, pp. 567-581] describe the computer coupled control of the glucose addition rate by monitoring the respiratory quotient (RQ = moles $CO_2$ produced/moles $O_2$ consumed) of yeast throughout the cultivation procedure, thus enabling precise control of glucose addition and specific growth rate so as to avoid the accumulation of ethanol.

Expression of recombinant proteins in yeast typically involves the use of extrachromosomal elements to harbor the DNA sequences required for the regulation and production of the desired polypeptide. Such expression systems generally employ bacterial-yeast "shuttle vectors" which contain the necessary genetic information to enable plasmid maintenance, segregation, and propagation of the heterologous gene sequence in both bacterial microorganisms and yeast [Bitter et al., (1987) *Methods in Enzymology,* vol. 152, pp. 673-684. Additionally, such expression vectors may employ the yeast 2μ replicon as a means of achieving high plasmid copy numbers. Alternatively, integration of the desired gene sequence into the yeast chromosome utilizing a translocation vector is also possible. Once transformed (or translocated) into yeast, regulation of expression of the heterologous DNA sequence can be either constitutive, enabling continuous production of the recombinant polypeptide while the yeast is viable, or by derepression or activation of some control element required to initiate mRNA synthesis of the DNA encoding the HBsAg gene sequence. Derepression or activation can be accomplished through the manipulation of some environmental parameter, such as temperature or the presence of light, or by alteration of the culture medium, through the addition, alteration, or deletion of some compound.

As mentioned previously, following expression in and purification from yeast, recombinant HBsAg as is currently used in Hepatitis B vaccines is found aggregated in a spherical particle with a 20 nm diameter. This particle is comprised of both proteins and lipids. Typically, these HBsAg-containing particles contain, by weight, about 80% HBsAg protein and about 20% yeast-derived lipid. These yeast-derived HBsAg particles produce consistently higher levels of antibody production in immunized individuals than are induced by intact Hepatitis B viral particles [Skelly et al., (1981) *Nature,* vol. 290, pp. 51-54]. Thus, current purification methods for yeast-derived HBsAg attempt to preserve the particle structure of yeast-derived HBsAg. To accomplish this, non-ionic detergents, found to be effective primarily to dissociate lipids from protein, without significant disruption of protein-protein associations, have been employed in lieu of ionic detergents because either use may cause the disassociation of protein complexes. For example, see PCT Patent Application No. US86/01704, which describes the use of the non-ionic detergent Triton X-100R in the extraction of recombinant HBsAg from yeast.

Because HBsAg is prepared as the immunogenic component of Hepatitis B vaccines for human immunization, after purification bulk HBsAg must be formulated into a composition suitable for human administration. Such formulations must preserve the immunogenicity of HBsAg over prolonged periods, meaning that the structure of the aggregated proteins must be maintained. In addition, excipients (inert macromolecules added to drug formulations), carriers (macromolecules to which a hapten is coupled), adjuvants (small molecules which augment cytokine formation and immune responses), etc. present in the formulation must meet the human administration guidelines of various worldwide and national health organizations and administrations.

As mentioned previously, two recombinant Hepatitis B vaccines, Recombivax HB® and Engerix-B®, have been approved by the FDA for human use in the United States. These vaccine formulations, manufactured by Merck Sharp & Dohme and Smithkline, respectively, consist of yeast-derived HBsAg bound to alum in an acceptable injectable buffer solution containing thimersol. Several other Hepatitis B vaccines, in addition to those described above, are in use in other countries, but have not yet been approved for use in the United States.

Through the utilization of an improved growth medium, culture conditions optimized for high yield HBsAg production, in conjunction with with an improved purification and adjuvanting procedure, a superior Hepatitis B vaccine formulation, i.e. one capable of promoting greater immune system priming than those vaccines currently available worldwide, can be developed utilizing recombinant DNA technology in yeast.

## SUMMARY OF THE INVENTION

One object of the present invention is to provide a novel Hepatitis B vaccine formulation suitable for mammalian administration comprising HBsAg and a bile acid salt. In one aspect of the invention, the amount of bile acid salt ranges from about 10 to about 30 percent by weight of the formulation. Particularly preferred is a formulation in which the bile acid salt represents about 20 percent by weight of the formulation. Preferred bile acid salts for use in accordance with the present invention are deoxycholates, particularly sodium deoxycholate. Additionally, the vaccine formulation described herein may further be comprised of about 2 to about 12 percent by weight lipid of yeast origin.

A second object of the present invention is to provide a method for developing a protective immunological response in mammalian species susceptible to Hepatitis B infection comprising administration of the disclosed vaccine formulation to such mammals. Of particular relevance is the administration of such a vaccine formulation to humans in accordance with the present invention.

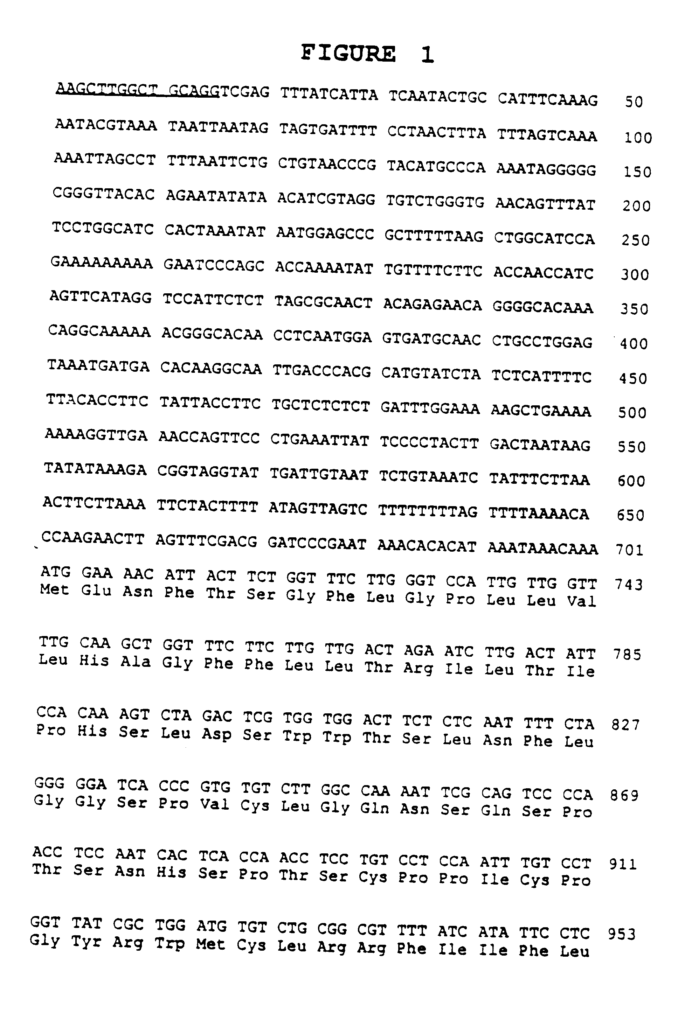

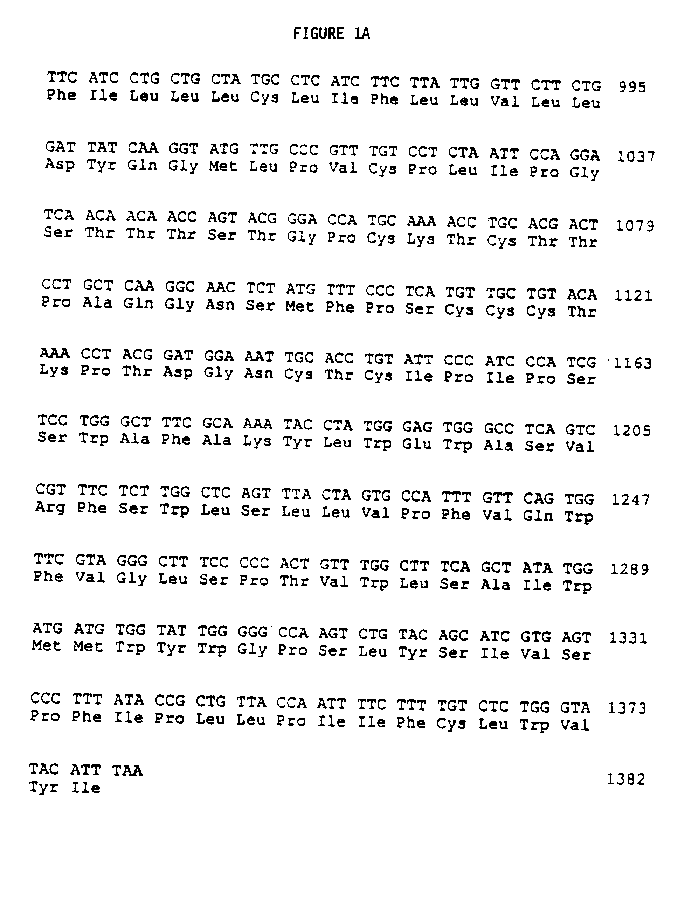

Another aspect of the present invention is that the HBsAg utilized in the vaccine formulation be of recombinant origin. Recombinant HBsAg derived as a result of recombinant yeast host cell expression is preferred, particularly when derived from recombinant *S. cerevisiae* employing the DNA sequence provided in Figure 1 [SEQ ID NO: 1].

Yet another aspect of the invention describes optimized carbon limited, low temperature growth conditions for yeast harboring recombinant plasmids, particularly *S. cerevisiae* harboring plasmids comprised of DNA sequences encoding HBsAg.

Still another aspect of the present invention details a method for the bulk purification of yeast-derived HBsAg wherein recombinant yeast, after expressing HBsAg, are lysed and HBsAg is bound to a colloidal silicate, particularly Aerosil 380, after which HBsAg is eluted, subjected to ion exchange chromatography, density gradient ultracentrifugation, and gel filtration chromatography utilizing a bile acid salt, particularly a deoxycholate, preferably sodium deoxycholate.

Additionally, purified bulk recombinant HBsAg vaccine material, comprising HBsAg and a bile acid salt, particularly a deoxycholate, e.g., sodium deoxycholate, may be adjuvanted by a method whereby the bulk vaccine material is added to a solution comprised of alum, the HBsAg binds to the alum, excess solution is removed, and adjuvanted vaccine preparation is resuspended in a buffer suitable for parenteral administration.

## BRIEF DESCRIPTION OF THE FIGURES

FIGURE 1 depicts the 1382 bp DNA fragment found in pGPD-1(HBS) that is responsible for the expression of HBSAg. This sequence includes 15 bp of DNA derived from m13mp9 (bp 1-15, underlined), the GPD portable promoter, the synthetic 5′ segment of the HBsAg gene (including 26 bp immediately upstream of the ATG) and the DNA sequence encoding HBsAg.

FIGURE 2 is a restriction map of pGPD-1(HBS).

Numerous aspects and advantages of the invention will be apparent to those skilled in the art upon consideration of the following detailed description which provides illumination of the practice of the invention in its preferred embodiments.

## DETAILED DESCRIPTION

The following description illustrates the practice of the present invention by detailing the construction of the recombinant vectors used to direct high level expression of HBsAg in yeast, novel procedures for achieving high cell density growth of transformed yeast harboring these DNA sequences, novel methods for purifying the resultant HBsAg such that Hepatitis B vaccine formulations incorporating this antigenic determinant, as manufactured and purified herein, may elicit greater protective immunity to HBV (all known serotypes) in immunized humans.

To achieve high level HBsAg expression in yeast, a plasmid, pGPD-1, comprising DNA replication origins from both *E. coli* and yeast, a highly efficient yeast promoter, yeast specific transcription termination/polyadenylation signals, and a selectable marker, was developed. The plasmid backbone is comprised of pBR322 having the entire 2μ plasmid (B form) from yeast inserted at the unique *Eco*RI site. This construction yields a plasmid capable of stable maintenance and propagation in both yeast and *E. coli.* When such a plasmid is transformed into a yeast strain such as *S. cerevisiae* RH218 (a cir° isolate lacking endogenous 2μ plasmid), it exhibits self-amplifying behavior. When the plasmid contains a suitable expression system, this self-amplifying behavior leads to increased synthesis of the desired heterologous product [Bitter et al., (1984) *Gene,* vol. 32, pp. 263-274; Bitter et al. (1987) *Methods in Enzymology*: *Recombinant DNA, Part D,* vol. 153, pp. 514-544]. Use of a host strain deficient in endogenous 2μ plasmid avoids potential recombination between the endogenous plasmid and the introduced recombinant plasmid.

The yeast GPD promoter was employed to direct the expression of the heterologous HBsAg gene. The GPD promoter was isolated from the glyceraldehyde-3-phosphate dehydrogenase gene. Termination/ polyadenylation signals are provided by those isolated from a yeast-derived TDH-3 gene. Construction of DNA vectors suitable for expression of heterologous proteins, particularly the 226 amino acid HBsAg polypeptide utilized in the present invention, in the yeast *S. cerevisiae* have been previously described. The recombinant plasmid encoding HBsAg preferred for use in accordance with the present invention is pGPD-1(HBS). See Bitter et al.(1984), *Gene,* supra; U.S. Patent No. 4,977,092, hereby incorporated by reference. Many procedures known to those skilled in the art have been developed to enable the introduction of foreign DNA into yeast, including transformation, transfection, microinjection, electroporation, and most recently by introduction of microscopic DNA-coated tungsten pellets fired into yeast by a 0.22 caliber blank. Thus, these or other alternative procedures may be used to introduce recombinant plasmids coding for high level HBsAg expression into yeast, particularly *S. cerevisiae.*

Once transformed, yeast harboring the recombinant plasmid(s) of the present invention may be grown under carbon-limited conditions in a medium providing sufficient nutrients to enable high cell densities and high level expression of the exogenous gene. Here, the recombinant product (HBsAg) is expressed constitutively. A wide variety of carbon sources, including glucose, glycerol, lactose, mannose, and molasses can be utilized by yeast. Cultivation can be continuous or by fed batch techniques over a temperature range of about 15 - 30°C. In addition, culture temperatures of less than 15°C may be used, although difficulties may be encountered in maintaining temperatures of less than 10°C in large scale fermentation vessels. The pH of the medium can range from about 43.5 to 7.5. In the instant invention, fed batch growth techniques are used.

After the cultivation has been completed, the yeast harboring the HBsAg are removed from the fermentation vessel. Because the desired product was not secreted, the cells, and not the cell broth, are retained for further processing. The cells are recovered by centrifugation or filtering. The resultant cell paste is then washed one or more times in an isotonic buffer. HBsAg may then immediately be purified from the cell paste or the paste may be stored at -20°C for future use.

Purification of HBsAg from yeast may be conducted as follows: Cell paste containing HBsAg produced in accordance with this invention may be initially resuspended in a solution containing non-ionic detergent. The resuspended cells are then passed through a cell disruption device to lyse the cells and release their contents, including soluble HBsAg, into the surrounding solution. Following cell lysis, the cell lysate is separated from the cellular debris, composed of membranes, unlysed cells, and other insoluble material, by centrifugation. The clarified HBsAg-containing lysate is then mixed with a colloidal silicate to which HBsAg will adsorb. After adsorption, the colloidal silicate (to which HBsAg is now bound) is separated from the solution by centrifugation or filtration. The resultant pellets are then rinsed several times to remove any unadsorbed material which may have come through the separation procedure. After rinsing has been completed, HBsAg is eluted from the colloidal silicate. This HBsAg-containing eluate is then subjected to ion exchange chromatography. The column effluent is collected, saved, and concentrated. Potassium bromide (KBr) is then added to this concentrated solution, which is then subjected to density gradient ultracentrifugation. The resultant gradients are then fractionated. Those fractions containing HBsAg are combined and a bile acid salt, preferably a deoxycholate, especially sodium deoxycholate, is added. This solution is then subjected to gel filtration chromatography in the presence of the deoxycholate. Those effluent fractions found to contain HBsAg are pooled. The resultant solution represents purified bulk HBsAg.

In addition, a large scale HBsAg purification process has also been developed and may be carried out as follows: HBsAg-containing cell paste is resuspended in a lysis buffer containing a non-ionic detergent. The cells in this homogenized slurry are then lysed, such as by subjection to mechanical shearing or high pressure. The lysate is clarified by diafiltration. The diafiltrate (which contains the HBsAg) is then mixed with a colloidal silicate to which HBsAg will adsorb. The HBsAg-adsorbed colloidal silicate is collected by filtration. HBsAg is then eluted from the colloidal silicate, and after filtration, to the filtrate is added a sufficient amount of affinity chromotography material to adsorb all HBsAg in the filtrate. This slurry is then filtered and buffer added to elute bound HBsAg. The eluate is collected, diafiltered, and concentrated. Potassium bromide density gradient ultracentrifugation is then performed. After fractionation of the resultant gradients, those fractions containing HBsAg are then pooled and a bile acid salt, such as a deoxycholate, preferably sodium deoxycholate, is added. This solution is then subjected to gel filtration. Fractions containing sufficiently purified HBsAg are collected and pooled and represent the purified bulk preparation containing 20 nm particles comprised of HBsAg.

Biochemical analysis of the 20 nm particles containing HBsAg after purification as described in the instant invention has revealed that these immunogenic particles are, by weight, comprised of about 80% protein (HBsAg), about 18% sodium deoxycholate, and about 2% yeast-derived lipid.

This bulk, purified preparation, containing from 5 μg/mL to 200 μg/mL HBsAg, is then converted into an adjuvanted formulation acceptable for human administration. Only adjuvants approved for use by the FDA are to be used if the resultant vaccine formulation is to be administered to humans. Aluminium hydroxide or aluminum phosphate, either of which is commonly referred to as alum, are two such FDA-approved adjuvants. The chosen adjuvant is added to the bulk purified HBsAg solution. This mixture is then centrifuged and the supernatant decanted. The resultant pellets are washed once. The washed pellets are then resuspended in a buffer suitable for human administration.

Alternatively, adjuvanting can be performed without the need to centrifuge the formulation. This can be accomplished adding purified bulk HBsAg to a solution suitable for mammalian administration so that the final HBsAg concentration is that needed for the particular vaccine. The solution to which the purified bulk HBsAg is added is comprised of a bile acid salt, like deoxycholate, and particularly sodium deoxycholate, alum, particularly aluminium hydroxide, and an appropriate physiological buffer. Adjuvanted, formulated HBsAg vaccine may then be packaged, or diluted and packaged, in accordance with approved FDA regulations. Immunization of patients with the HBsAg-based HBV vaccine may then be conducted.

The following examples are offered to more fully illustrate the present invention. In addition, the Examples provide a preferred embodiment of the present invention but are not meant to limit the scope thereof.

EXAMPLES

EXAMPLE 1

Expression Vector Construction

To direct the expression of heterologous genes in *S. cerevisiae,* a yeast GPD promoter was chosen. Holland et al. [(1978) *Biochemistry,* vol. 17, pp: 4900-07; (1980) *J. Biol. Chem.,* vol. 255, pp. 2596-2605; and (1983) *J. Biol. Chem.,* vol. 258, pp. 5291-99] isolated and sequenced each of three non-tandemly repeated structural genes for glyceraldehyde-3-phosphate dehydrogenase found in *S. cerevisiae.* GPD accounts for up to 5% of the dry weight of commercial bakers yeast and the mRNA encoding this enzyme represents 2 - 5% of total yeast polyadenylated mRNA [Holland et al., (1978) *Biochemistry,* supra]. One of the three isozymes is much more abundant than the others [Jones et al., (1972) *FEBS Lett.,* vol. 22, pp. 185-9]. The gene encoding this most abundant isozyme is designated pgap491 [Holland et al., (1979a) *J. Biol. Chem.,* vol. 254, pp. 5466-74]. Bitter et al. [(1984) *Gene,* supra] isolated and sequenced the promoter for this particular GPD isozyme.

For use in the present invention, the functional GPD promoter was initially isolated from pp6y [Musti et al., (1983) *Gene,* vol. 25, pp. 133-43] as a 2.1 kb (kilobase) *Hind*III fragment. After this fragment was purified by agarose gel electrophoresis, it was excised from the gel and the DNA isolated and purified. The DNA was then subjected to *Taq*I digestion. After digestion, polyacrylamide gel electrophoresis was used to separate the 653 bp (base pair) fragment containing the GPD promoter from the other digestion products. The region of the gel containing the this fragment was then excised and the fragment eluted. This GPR promoter-containing fragment was then cloned into M13mp9 which had been previously digested with AccI and treated with alkaline phosphatase to prevent religation of uninserted vector. After transformation and outgrowth in *E. coli* strain JM109, RF (replicative form) DNA was purified from isolated plaques which had been grown up. RF DNA from a resultant M13 clone found to harbor GPD in the correct orientation (i.e. having the GPD transcription start site adjacent to the *Bam*HI site in the phage's polylinker) was then digested with *Bam*HI and *Hind*III. After agarose gel electrophoresis, the fragment containing the GPD promoter was excised from the gel and then DNA isolated and purified.

Similarly, the pBR/2μ plasmid construct was digested with *Bam*HI and *Hind*III. Digested vector can be separated from the 346 bp pBR322 insert by electrophoresis, followed by excision, isolation, and purification. Alternatively, the restriction products can be treated with alkaline phosphatase, followed by phenol extraction, to prevent vector religation to the *Bam*HI/*Hind*III insert. In either instance, the resultant vector DNA preparation is ligated to *Bam*HI/*Hind*III GPD promoter-containing fragment. The resultant vector is designated pGPD.

To complete the construction of the expression vector, designated pGPD-1, used to produce the HBsAg employed in the present invention, the yeast TRP1 gene was included. The TRP1 gene provides a selectable marker following transformation as well as providing efficient termination and polyadenylation signals to yeast RNA polymerase II. The TRP1 gene utilized was an 852 bp *Eco*RI/*Bgl*II fragment isolated and purified as described by Tschumper and Carbon [(1980) *Gene,* vol. 10, pp. 157-66]. Previously, Amerer et al. [(1981) *Proceedings of the Third Cleveland Symposium on Macromolecules: Recombinant DNA.* Elsevier, Amsterdam, pp. 185-97] demonstrated that in an expression vector construction comprising a heterologous gene followed by the TRP1 gene (in an orientation such that the resultant mRNA is comprised of the sense strands for both genes, 5′ to 3′), the TRP1 gene provided efficient transcription termination and polyadenylation signals after read through of the heterologous gene.

In preparation for the ligation of the *Eco*RI/*Bgl*II TRP1 gene fragment, pGPD was digested with *Bam*HI, followed by alkaline phosphatase treatment and subsequent phenol extraction. A ligation reaction containing appropriate quantities of *Bam*HI-cleaved, phosphatase treated vector, purified 852 bp *Eco*RI/*Bgl*II TRP1 fragment, and a phosphorylated *Bam*HI/*Eco*RI linker, was conducted. This ligation resulted in the reconstitution of the *Bam*HI site adjacent to the GPD transcription termination signal and of the EcoRI site 5′ of the TRP1 gene. However, the annealing of the 5′ overhangs of the *Bam*HI and *Bgl*II sites 3′ to the TRP1 gene eliminated these recognition sites for both *Bam*HI and *Bgl*II. Following ligation and bacterial transformation, plasmid DNA from various clones was subjected to restriction analysis to determine which clones contained plasmids having the TRP1 gene in the correct orientation relative to the GPD promoter. Clones containing TRP1 in the proper orientation were designated pGPD-1.

As previously discussed, HBsAg is a polypeptide comprised of 226 amino acids. The gene encoding this polypeptide is included on a *Bam*HI fragment approximately 1,400 bp in size in the HBV serotype adw genome [Valenzuela et al., (1979) *Nature,* vol. 280, pp. 815-19]. This *Bam*HI fragment includes 126 bp of non-coding DNA 5′ to the protein's translation initiation codon as well as 573 bp of 3' untranslated HBV DNA and 23 bp of pBR322 DNA (a cloning artifact). After *Bam*HI digestion and fragment separation by agarose gel electrophoresis, the 1,400 bp HBsAg-encoding fragment was excised and the DNA isolated and purified.

The yeast shuttle vector YRp7 [Struhl et al., (1979) *Proc. Natl. Acad. Sci. USA,* vol. 76, pp. 1035-39] was also digested with *Bam*HI. Following BamHI digestion, YRp7 was treated with alkaline phosphatase and then phenol extracted. *Bam*HI cleaved YRp7 was then ligated to the 1,400 bp *Bam*HI fragment coding for HBsAg, creating plasmid pHBsAg. To remove the non-coding 3′ HBV and pBR322 DNA, pHBsAg was subjected to a complete *Hpa*1 digestion, followed by a partial *Hind*III digestion. The vector was then treated with the Klenow fragment of *E. coli* DNA polymerase 1 to generate blunt ends. This DNA mixture was then ligated to itself and those clones having the pBR and HBV 3′ non-coding sequences deleted were designated pHBs.

Additionally, the non-coding HBV DNA 5′ to the HBsAg initiation codon was deleted by digesting pHBs with *Bam*HI and *Xba*I. This 218 bp fragment also contained the coding information for the first 32 amino acids of HBsAg. To restore this coding information, a synthetic *Bam*HI/XbaI fragment was generated. The DNA sequence of this synthetic fragment is described in Figure 1 [SEQ ID NO: 1]. The synthetic fragment not only eliminates nonessential 5′ untranslated DNA; the 5′ untranslated portion of the synthetic fragment was also designed to be A-rich and G-deficient, a characteristic Amerer et al. [(1981) supra] identified as important in efficient translation initiation in highly expressed yeast proteins. The 27 bp immediately 5′ to the ATG corresponds to the 5′ untranslated GPD leader described by Holland et al. [(1979) *J. Biol. Chem.,* vol. 254, pp. 9839-45]. Further, in that portion of the synthetic DNA fragment which provide amino acid coding information, codons preferentially utilized in highly expressed yeast genes, as disclosed by Bennetzen et al. [(1981) *J. Biol. Chem.,* vol. 257, pp. 3026-31], were incorporated. Plasmids containing the synthetic *Bam*HI/*Xba*I fragment were designated pHBs-2.

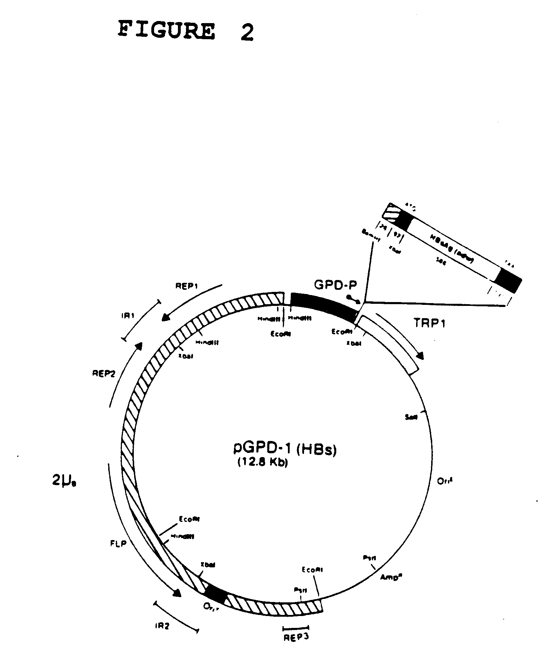

pHBs-2 was then digested with *Bam*HI and *Eco*RI so the fragment encoding HBsAg could be isolated. Following *Bam*HI/*Eco*RI digestion, the sample was subjected to S1 nuclease treatment to generate blunt ends. The blunt ended 850 bp fragment encoding HBsAg (see Figure 1 [SEQ ID NO: 1] for entire coding sequence) was then separated from other digestion products by agarose gel electrophoresis. The DNA encoding HBsAg was then excised and purified. pGPD-1 was cleaved with *Bam*HI and also blunted with S1 nuclease, followed by phosphatase treatment and phenol extraction. The S1 blunted 850 bp HBsAg-encoding fragment was then ligated into the *Bam*HI digested, S1 treated pGPD-1 to create pGPD-1(HBs), which was then introduced into *S. cerevisiae* RH218. A restriction map for pGPD-1(HBS) is shown in Fig. 2.

EXAMPLE 2

Growth Method for HBsAg-containing Yeast

The following description details a production method for recombinant HBsAg employing a 400 L fermentation vessel wherein the *S. cerevisiae* strain RH218 harboring the HBsAg-containing expression vector (pGPD-1(HBS)) is cultivated at about 25°C under carbon limited conditions. All procedures described herein are performed aseptically unless otherwise indicated. Unless otherwise stated, all sterilization procedures used throughout this example are conducted by heating the apparatus or solution to be sterilized to 121 - 123°C for 30 ± 5 min.

An initial seed culture is prepared by inoculating each of eight inoculating flasks containing 500 mL YMS (Yeast Minimal Salt) with 500 μl RH218 pGPD-1 kept as a frozen glycerol stock. These cultures are then placed on a rotary shaker at 275 ± 25 RPM at 30 ± 2°C for 40 - 50 hours. When these cultures reach an $OD_{600}$ of about 4 - 6, they are pooled and transferred to a 400 L fermentation vessel which has been previously prepared as follows.

As the seed cultures are growing, the fermentation media is prepared as follows. 320 g glucose, 3.2 g inositol, and 640 mL of 1 M $MgSO_4 \cdot 7H_2O$ are dissolved in $H_2O$ and brought to a final volume of 3.33 L. Following dissolution, this solution is sterilized. Upon cooling, 480 mL of Trace Metals solution (Table 1), 480 mL of Vitamin solution (Table 2), and 64 mL of filter sterilized 1% (w/v) Thiamine solution are added.

TABLE 1

| TRACE METALS SOLUTION | |
|---|---|
| Compound | g/l |
| $FeCl_3 \cdot 6H_2O$ | 27.0 ± 0.3 |
| $ZnCl_2$ | 2.0 ± 0.03 |
| $CoCl_2 \cdot 6H_2O$ | 2.0 ± 0.03 |
| $NaMoO_4 \cdot 2H_2O$ | 2.0 ± 0.03 |
| $CaCl_2 \cdot 2H_2O$ | 1.0 ± 0.02 |
| $CuSO_4 \cdot 5H_2O$ | 1.9 ± 0.03 |
| $H_3BO_3$ | 0.5 ± 0.01 |
| $MnCl_2 \cdot 4H_2O$ | 1.6 ± 0.03 |
| Sodium Citrate·$2H_2O$ | 73.5 ± 1.0 |

[prepare by dissolving the ingredients in about 90% of total lot volume with purified $H_2O$; after dissolution, adjust to desired final lot volume with purified $H_2O$; sterilize by filtration though a 0.2 μm filter]

TABLE 2

| VITAMIN SOLUTION | |
|---|---|
| Compound | g/l |
| Biotin | 0.06 ± 0.001 |
| Folic Acid | 0.04 ± 0.001 |
| Pyridoxine | 1.4 ± 0.03 |
| Riboflavin | 0.42 ± 0.008 |
| Pantothenic Acid | 5.4 ± 0.11 |
| Niacin | 6.1 ± 0.12 |
| Compound | mL/l |
| 10 N Sodium Hydroxide | 5.31 ± 0.11 |

[prepare by. (a) dissolving Biotin, Folic Acid, and Riboflavin in about 4% of total lot volume of purified $H_2O$ and 5.65 ± 0.19% of total lot volume 10 N NaOH; after dissolution, adjust to 5% of total lot volume with purified $H_2O$; (b) dissolve Pyridoxine and Niacin in about 2.0% of total lot volume of purified $H_2O$ and 94.2 ± 0.19% of total lot volume of 10 N NaOH; after dissolution, adjust to 2.5% of total lot volume with purified $H_2O$; (c) dissolve Pantothenic Acid in about 2.0% of total lot volume of purified $H_2O$ and 0.188 ± 0.019% of total lot volume of 10 N NaOH; after dissolution, adjust to 2.5% of total lot volume with purified $H_2O$; (d) combine the three solutions and adjust to total lot volume with purified $H_2O$; (e) sterilize by filtration though a 0.2 μm filter]

Approximately 140 L of $H_2O$ are added to a 400 L fermenter. 4040 g Casamino acids, 2160 g $KH_2PO_4$, 600 g $(NH_4)_2SO_4$, and 10 mL Dow P2000 Antifoam are then added to the fermentation vessel. The fermenter and its contents are then sterilized. After sterilization, the fermenter's temperature controller is set to 25°C (fermenter contents to be maintained at 25 ± 2°C). The 3.33 L of fermentation media is then added to the vessel. The pH of the resultant solution is adjusted to 4.5 ± 0.2 (pH controller set to 4.5) using $H_3PO_4$ or $NH_4OH$. The aeration rate is set at 160 ± 16 slpm (liters per minute) and back pressure is adjusted to 0.34 ± 0.07 bar. Agitation initially is 200 ± 20 RPM and the dissolved oxygen (DO) amplifier is adjusted to 100 ± 5%.

Off gas is monitored so the Respiratory Quotient (RQ; moles of $CO_2$ produced/moles of $O_2$ consumed)

can be determined. The culture will initially consume the glucose in the media and partially metabolize it to ethanol. After the glucose is depleted, the culture will metabolize any ethanol present. An RQ less than 1 indicates the culture is metabolizing ethanol. An RQ of 1 to 1.1 indicates that glucose is being completely metabolized to $CO_2$ and $H_2O$. An RQ greater than 1.1 indicates that partial oxidation of glucose to ethanol is occurring. After Feed Medium addition begins, the RQ should be maintained below 1.1 to prevent ethanol accumulation. The RQ can be adjusted by lowering the Feed Medium addition rate until the RQ falls into the desired range.

60 L of Feed Medium is prepared in a sterilized stirred feed vessel by adding 48 kg of glucose, 3.0 g inositol, and 1350 mL of 1 M $MgSO_4 \cdot 7H_2O$ in sufficient water to allow dissolution in the presence of heat and stirring. After complete dissolution, this solution is sterilized. Additionally, a 30 L solution comprised of 9. 84 kg Casamino acids, 257 g $KH_2PO_4$, and 450 g $(NH_4)_2SO_4$ is prepared and sterilized for 60 ± 10 min. After both solutions have cooled to room temperature, 129 mL of filter sterilized 1% (w/v) Thiamine solution, 608 mL Trace Metals solution, and 608 mL Vitamin solution are added to the 30 L Casamino acid-containing solution. Following thorough mixing, this solution is then combined with the 60 L Feed Medium solution. The Feed Medium vessel is then connected to the fermenter.

As the fermentation progresses, the pH is maintained at 4.5 ± 0.2 and the temperature is kept at 25 ± 2°C. DO is maintained at about 50% saturation by increasing the air flow, agitation, and/or back pressure. DO must not be allowed to drop below 25% at any time. The culture's $OD_{600}$ is monitored every 4 hours. Feed Medium is added at the flow rate designated in Table 3.

TABLE 3

| $OD_{600}$ | Flow Rate (mL/hr) |
|---|---|
| 1.4 | 67 ± 7 |
| 2.8 | 200 ± 20 |
| 5.5 | 400 ± 40 |
| 8.1 | 600 ± 60 |
| 14.0 | 800 ± 80 |
| 18.0 | 1000 ± 100 |
| 35.0 | 1200 ± 120 |
| 40.0 | 1600 ± 160 |
| 45.0 | 1800 ± 180 |
| 55.0 | 2200 ± 220 |
| 60.0 | 2600 ± 260 |
| 65.0 | 3000 ± 300 |
| 70.0 | 3400 ± 340 |
| 75.0 | 3600 ± 360 |
| 86.0 | 4000 ± 400 |

Additionally, the RQ is monitored hourly. If the RQ is found to exceed 1.1, the Feed Medium flow rate is reduced to the next lower level. If the RQ is below 1.1, the Feed Medium flow rate is increased to the next level (however, the flow rate should not exceed that listed in Table 3 for any given $OD_{600}$).

When the $OD_{600}$ becomes greater than 80 and less than 120, the fermentation is halted by cooling the fermentation vessel to 5 - 15°C and by discontinuing Feed Medium addition. Also, agitation is stopped so the culture' volume can be estimated. After an estimation as to the culture's final volume is made, agitation at 200 - 250 RPM is resumed and the fermenter is connected with an ultrafiltration unit so as to produce a closed system. The ultrafiltration unit is equipped with filter membranes having a 100,000 molecular weight cut-off.

The culture is then concentrated to 50 ± 5% of its estimated final volume using this system. Following this initial two-fold concentration, sufficient Yeast Cell Wash Buffer (YCWB; 50 mM Tris, 10 mM EDTA, pH 8.0) is added to restore the culture's estimated final volume. This washing procedure is repeated two additional times.

Finally, the culture is concentrated to 50 ± 5% its estimated final volume. The concentrated culture is then removed in aliquots from the fermenter and loaded into 1 L centrifuge bottles containing sample bags for concentration into cell paste. The bottles are spun at 4,000 ± 200 RPM at 4 ± 2°C for 20 ± 2 min. Supernatants are discarded and the cell pellets weighed. After weighing, the pellets are stored at -40°C or colder until they are to be subjected to HBsAg particle purification.

## EXAMPLE 3

### Alternative Growth Method for HBsAg-containing Yeast

The following description details an alternative production method for recombinant HBsAg employing a 400 L fermentation vessel wherein the *S. cerevisiae* strain RH218 harboring the HBsAg-containing expression vector (pGPD-1(HBS)) is cultivated at low temperature under carbon limited conditions. All procedures described herein are performed aseptically unless otherwise indicated. All sterilization procedures used throughout this example are conducted by heating the apparatus or solution to be sterilized to 121 - 123°C for 30 ± 5 min.

An initial seed culture is prepared by inoculating each of eight inoculating flasks containing 500 mL YMS with 500 μl RH218 pGPD-1 kept as a frozen glycerol stock. These cultures are then placed on a rotary shaker at 275 ± 25 RPM at 30 ± 2°C for 40 - 50 hours. When these cultures reach an $OD_{600}$ of about 4 - 6, they are pooled and transferred to a 400 L fermentation vessel which has been previously prepared as follows.

117 ± 6 L of $dH_2O$ is added to the fermenter. 3030 g of Casamino acids, 1620 g of $KH_2PO4$, 450 g of $(NH_4)_2SO_4$, and 10 ml of Dow P2000 Antifoam are then added to the vessel. The fermenter and its contents are then sterilized. After sterilization, the vessel is cooled to 25 ± 2°C and the fermentation temperature controller is set at 25°C. Concentrated fermentation medium, described as follows, is then added to the fermenter.

Concentrated fermentation medium is prepared by combining about 240 g of glucose, about 2.4 g inositol, and about 118 g $MgSO_4{\cdot}7H_2O$ in $dH_2O$ until a final volume of 1 L is achieved. This solution is then sterilized. After the solution has cooled to near room temperature, 360 mL of Trace Metals solution (see Example 2, Table 1), 48 mL of a 1% Thiamine solution, and 360 mL of Vitamin solution (see Example 2, Table 2) is then added. After mixing, this solution is added to fermenter as described above.

The fermenter contents are next adjusted to pH 4.5 ± 0.2 with $NH_4OH$ or $H_3PO_4$ under the control of the pH control which is set at pH 4.5. The vessel's aeration rate is set at 160 ± 16 slpm with a back pressure of 0.34 ± 0.07 bar. Agitation is set at 200 ± 20 RPM and the dissolved oxygen (D.O.) amplifier is set at 100 ± 5%. Additional sterilized Dow P2000 is also attached to the fermentation vessel.

The pooled seed cultures are then transferred to the fermenter. The initial $OD_{600}$ of the fermentation culture is about 0.1. The Respiratory Quotient is calculated and used to adjust the feed rate so as to minimize ethanol formation. Initially, the culture's pH should be maintained at 4.5 ± 0.2 and the temperature should be held at 25 ± 2°C. In addition, the dissolved oxygen (D.O.) level should be kept above 50% saturation through a combination of increasing agitation, air flow rate, oxygen flow, and back pressure (back pressure should never exceed 1.0 bar). D.O. should never fall below 25% saturation. Optical density, R.Q, and ethanol concentration should be monitored every four hours throughout the course of the fermentation.

After the fermentation has been initiated, the Feed Medium is prepared as follows. In a stirred feed vessel, Feed Medium 1 (FM1) is prepared by dissolving, in the presence of stirring and heat, 123 kg glucose, 7.7 g inositol, and 850 g $MgSO_4{\cdot}7H_2O$ in sufficient $dH_2O$ to achieve a final volume of 160 L. After complete dissolution of these compounds, FM1 is sterilized. Following sterilization, FM1 is cooled to about room temperature. 330 mL of a 1% Thiamine solution, 1550 mL of Trace Metals solution, and 1550 mL of Vitamin solution is added. Feed Medium 2 (FM2) is prepared in another stirred feed vessel by adding 25.2 kg Casamino Acids, 657 g $KH_2PO_4$, and 1150 g $(NH_4)_2SO_4$ to sufficient $dH_2O$, in the presence of stirring and heat, to achieve a final volume of 70 L. Upon dissolution of all the FM2 ingredients, this solution is sterilized. After cooling to near room temperature, FM2 is mixed into FM1. The vessel containing this Feed Medium is then attached to the fermentation vessel.

When the $OD_{600}$ of the fermentation culture reaches 0.8 and the ethanol (EtOH) concentration is less than 0.5 g/l, addition of the Feed Medium is initiated at an initial rate of 50 mL/hour. The feed rate is adjusted according to the schedule in Table 4.

TABLE 4

| Adjusted Feed Rate | |
|---|---|
| EtOH (g/l) | (F = previous feed rate) |
| EtOH > 2.0 | 0.75 x F |
| 1.0 < EtOH ≦ 2.0 | 0.80 x F |
| 0.5 < EtOH ≦ 1.0 | 0.90 x F |
| 0.2 < EtOH ≦ 0.5 | F |
| EtOH ≦ 0.2 | 1.20 x F |

In the event EtOH measurement is not possible, the following schedule may be used.

| Adjusted Feed Rate | |
|---|---|
| RO | (F = previous feed rate) |
| RQ > 1.20 | 0.75 x F |
| 1.10 < RQ ≦ 1.20 | 0.80 x F |
| 1.05 < RQ ≦ 1.10 | 0.90 x F |
| 0.90 < RQ ≦ 1.05 | 1.20 x F |
| RQ ≦ 0.90 | F |

When an $OD_{600}$ of greater than or equal to 5.0 is reached, the temperature set point is lowered to 15°C and the pH set point is adjusted to 5.5. pH should be maintained at 5.5 ± 0.2 and the culture temperature should be kept at 15 ± 2°C. When a total fermentation volume of 300 L is obtained, liquid is drawn off to reduce the total culture volume to 250 L. 30 ± 6 hours after an $OD_{600}$ = 90 ± 5 is achieved, the fermentation is terminated by turning off the Feed Medium and the air/oxygen supply.

Agitation is discontinued and the final culture volume is estimated. After the final volume is estimated, agitation is resumed at 120 ± 20 RPM. Utilizing a previously sanitized closed ultrafiltration system (Romicon) containing 0.1 μm or larger cut off filter membranes connected to the fermenter, the cell broth is concentrated to 50 ± 5% of its original volume. Alternatively, a Millipore System employing a membrane having a 0.45 μm pore size can be used to concentrate the cell broth. After concentration, the culture volume is increased to its original volume ± 5% through the addition of previously prepared sterile 50 mM EDTA, pH 8.0 buffer. The concentration and buffer addition is conducted an additional four times.

Finally the cell broth is concentrated to 40 ± 5% of its original volume, with the concentrated culture being returned to the fermentation vessel. The concentrated culture is agitated at 150 ± 50 RPM. The $OD_{600}$ is measured, agitation is briefly interrupted to obtain an estimated final concentrated broth volume. The fermenter and its contents are then rapidly chilled. After chilling, the concentrated broth is dispensed into sample bags. The sample bags are then weighed. After weighing, the samples are stored between -40 and -80°C until they are processed for HBsAg.

## EXAMPLE 4

### HBsAg Purification

The following example embodies the procedures used in a typical purification of HBsAg as produced in Example 2. Because the HBsAg so produced is to be used in a human vaccine formulation, the procedures described herein are performed in a manner to insure that the final preparation is sterile and pyrogen free. Such procedures are common and known to those skilled in the art of pharmaceutical manufacturing. Unless otherwise indicated, at no time during the purification process should the temperature of the HBsAg-containing solution exceed 10°C. Additionally, unless otherwise specified, all mixing procedures described herein are conducted at 5 ± 3°C using an overhead mixer, such as a Brinkman homogenizer, set at a speed so as to minimize solution splashing and foaming but fast enough so as to prevent settling out of solution components.

(a) Cell Lysis

4,700 ± 50 g of HBsAg-containing cell paste, as produced in Example 2, is combined with 5,000 ± 50 mL of 2X Lysis Buffer (0.1 M Tris-HCl, 0.02 M EDTA, 10% [v/v] glycerol, 0.5% [v/v] Triton X-100, pH 8). The solution volume is then adjusted to 9950 + 50 mL using water for injection (WFI). The volume of the solution at this point must not exceed 10,000 mL. The solution temperature should be maintained at 5 ± 3°C by placing the vessel containing the cell solution in a water bath of appropriate temperature. :he cell paste is then completely resuspended using an overhead mixer. After resuspension, the $OD_{600}$ of the solution is measured in triplicate. The average solution $OD_{600}$ should be between 75 and 125.

Following resuspension, the pH of the solution is adjusted to pH 7.9 ± 0.2 using 2 M Tris base. Solution temperature should be maintained at 5 ± 3°C. This solution is then passed through a cell disruption device, such as a Dyno Mill (Glen Mills, Inc., Maywood, NJ), to lyse the cells. The Dyno Mill pump flow rate should be 3,700 ± 100 mL/hr. and the Dyno Mill chiller set for -20 to -30°C. Initially, the cell suspension is pumped without agitation into the Dyno Mill agitation chamber. As material begins to emerge from the chamber outlet, agitation is started at a shaft speed of 3,350 RPM and an agitator disc peripheral speed of 14 m/sec. The first 200 ± 25 mL of cell lysate to emerge from the chamber after agitation is initiated is collected and added back to the unlysed cell suspension. All additional cell lysate to come out of the agitation chamber is recovered in ice cooled collection vessels in 5 ± 1 L aliquots. After the last of the cell suspension material has entered the Dyno Mill inlet tubing, the flow pump is turned off. The inlet tubing is then disconnected from the cell suspension container and reconnected to a vessel holding 500 ± 50 mL of 1X Lysis Buffer. The flow pump is then again turned on and run until the last of the 1X Lysis Buffer has been drawn into the inlet tubing, at which point the collection of cell lysate is discontinued. The volume of cell lysate in each collection vessel is then measured.

(b) Clarification

Next, the cell lysate is clarified by centrifugation as follows. The cell lysate is transferred to 1 L polypropylene centrifuge bottles and loaded into a Beckman JS 4.2 rotor. These samples are then spun in a Beckman J6-B centrifuge (chamber temperature 0°C) at 4,100 ± 100 RPM for 120 ± 5 min. Following centrifugation, the supernatants are carefully decanted and pooled, after which the final volume of the clarified lysate is measured. A 25 μl aliquot of this clarified lysate is removed and subjected to a protein assay to determine the total protein content in the solution. One assay useful in this context includes diluting the sample 100-fold in WFI and subjecting it to a coomassie blue binding assay (commonly referred to as a Bradford assay).

(c) Adsorption

After the clarified lysate's total protein content is ascertained, that figure is multiplied by 0.75 to yield the amount of Aerosil 380 needed for complete HBsAg adsorbtion. Using a mixer, the calculated amount of Aerosil is stirred into the clarified lysate over 5 - 10 min. Once the Aerosil addition has been completed, stirring is continued for an additional 8 - 16 hr in a cold room at 5 ± 3°C to allow protein adsorbtion to occur.

Following the adsorbtion procedure, the Aerosil-containing suspension is transferred to 1 L polypropylene bottles and centrifuged at 4,100 + 100 RPM in a J6-B (chamber temperature 0°C) in a JS 4.2 rotor for 10 - 15 min. The supernatants are carefully decanted so as not to disturb the pellets, which are retained. The pellets are extracted from each bottle and combined in an appropriate container. A volume of TNE (20 mM Tris, 150 mM NaCl, 5 mM EDTA pH 8) is measured out equal to that of the clarified lysates following centrifugation. A small amount of this TNE is then used to rinse out each centrifuge bottle from which protein-adsorbed Aerosil was removed. These rinses are combined in the same container as that holding the pellets. The remainder of the TNE is then added to this container. In a coldroom (5 ± 3°C), these pellets are resuspended. After resuspension has been completed, mixing is continued an additional 20 ± 10 min. The solution is again centrifuged as above, the pellets are retained and resuspended in TNE. This TNE rinse of protein-adsorbed Aerosil is conducted for a third time as well, utilizing the same procedure as described above. However, following the centrifugation step, the pellets, which are again retained, are subjected to an elution procedure to release the adsorbed protein.

(d) Elution

The elution procedure is conducted by collecting the protein adsorbed, Aerosil-containing pellets into an appropriate container. A volume of Elution Buffer (10 mM sodium borate, 1 mM EDTA, 0.25% [w/v] sodium deoxycholate, pH ~ 9.25) equal to twice the volume of the clarified lysate is measured out. A small volume of the Elution Buffer is used to rinse the centrifuge bottles from which the pellets to be resuspended were removed. These Elution Buffer rinses, along with the rest of the Elution Buffer that had been measured out, are then added to the container into which the pellets were transferred. Again in a coldroom (5 ± 3°C), an overhead mixer is used to resuspend the pellets. When no Aerosil clumps remain evident in the solution, but while mixing is continued, the solution's pH is adjusted to 9.25 ± 0.05 using 10 N NaOH. Following this pH adjustment, mixing is continued another 75 ± 10 min.

This slurry, after being loaded into 1 L polypropylene centrifuge bottles, is subjected to centrifugation in a J6-B (chamber temperature 0°C) at 4,100 ± 100 RPM for 25 - 30 min. in a JS 4.2 rotor. The resultant supernatants, which contain protein eluted from the Aerosil, are carefully decanted and saved. The pellets are discarded. The supernatants are pooled and the pH of this solution is adjusted to 8.0 + 0.1 using 2 N HCl. The volume of this pH adjusted Aerosil eluate solution is then measured and designated volume A. This eluate solution is then stored at 5 ± 3°C until ion exchange chromatography is conducted.

(e) <u>Ion Exchange Chromatography</u>

A DEAE-cellulose column prepared as follows. A DEAE-cellulose column having a bed volume of 6750 ± 200 mL for each 10 L of starting cell suspension is prepared. The DEAE column Buffer is 20 mM Tris-HCl, 0.02% (w/v) sodium metabisulfite, 5 mM EDTA, 0.11 M NaCl, pH 8. The column is operated in a cold room at 5 ± 3°C. Column effluent is spectrophotometrically monitored at 275 - 285 nm and 1 - 2 AUFS. A chart recorder, set at a speed of 0.1 - 0.2 mm/min. at a range of 10 millivolts (mv), is attached to spectrophotometer to record spectral absorbance over time. Once the column bed has been prepared, DEAE Column Buffer is pumped through the column until a stable UV absorbance baseline is established. Prior to loading the pH adjusted Aerosil eluate onto the DEAE-cellulose column, the following solutions (each having a temperature of 4 ± 4°c) are mixed into the eluate solution in quantities calculated by the following formulas:

i) A x 0.0084 = volume of 0.5 M EDTA, pH 8 to add;
ii) A x 0.021 = volume of 1 M Tris-HCl, pH 8, to add; and
iii) A x 0.0021 = volume of 10% (w/v) sodium metabisulfite to add.

Once these solutions have been added to the Aerosil eluate, the solution's pH is adjusted to pH 8.02 ± 0.03 using 10 N NaOH or 2 N HCl. Additionally, the solution's conductivity is adjusted to 14.1 ± 0.3 mmho/cm (as measured at 22 ± 2°C using a conductivity meter) by adding approximately 5 M NaCl per 10 L of Aerosil eluate prior to the addition of EDTA, Tris-HCL, and sodium metabisulfite.

Following the solution conductivity adjustment, all DEAE Column Buffer above the bed surface is removed and the Aerosil eluate solution is then carefully loaded onto the DEAE-cellulose column. Once a sufficient volume of sample has been loaded, such as when the the surface of the sample solution is more than 10 cm above the top of the column bed, column flow continues at the same rate as was used earlier to pump DEAE Column Buffer through the column. Sample loading continues so as to maintain the desired space between the solution's surface and the top of the column bed. Column effluent is monitored by UV. The initial effluent, equal to approximately 30% of the column bed volume, is discarded. After this point, all effluent coming off the column is collected and saved. Once all the protein-containing solution has been loaded onto the column, the column is washed with an amount of DEAE Column Buffer equal to 1.4 times (± 400 mL) the column bed volume. All column effluent is collected in the same container. Following effluent collection, the effluent may stored at 5 ± 3°C for up to 12 hr before being concentrated.

Effluent concentration is conducted by using a Millipore Pellicon ultrafiltration unit employing 2 cassettes of polysulfone membranes having 100,000 dalton molecular weight cut offs (Millipore cassette PTHK 00005) that had previously been equilibrated and purged of air by twice passing 2,000 ± 500 mL of DEAE Column Buffer through the unit at a peristaltic pump rate 500 ± 200 mL/min. During the concentration procedure, all solutions should be maintained at a temperature of 4 ± 4°C. The hold-up volume of the concentration unit (internal volume of the membrane cassettes and tubing) should not exceed 250 mL. During effluent concentration, the pump flow rate (filtrate rate plus retentate rate) should be about 1,000 mL/min, with a filtrate rate of 280 ± 50 mL/min. and a retentate rate of 720 ± 50 mL/min. Once the effluent volume has been concentrated to 4.0 ± 0.2 L (including the hold-up volume), concentration is stopped and 420.0 ± 0.5 g KBr per liter is added to the concentrated effluent and mixed until dissolved. In addition, a solution comprised of 126 ± 1 g KBr dissolved in 300 ± 3 mL DEAE Column Buffer is prepared separately and stored at 5 ± 3°C. After the KBr has dissolved in the concentrated effluent solution, concentration is continued further, but at a decreased flow rate of approximately 500 mL/min (filtrate rate of 140 ± 25 mL/min; retentate rate of 360 ± 25 mL/min) until the effluent's volume is reduced to 1.5 ± 0.2 L. At this point, the pump flow rate is further reduced to about 250 mL/min (filtrate rate of 40 ± 6 mL/min; retentate rate of 210 ± 9 mL/min). Concentration is continued until a 350 ± 10 mL volume (excluding the hold-up volume) is achieved.

At this point, the concentrated KBr-containing effluent is recirculated through the membrane cassette system for 5 ± 2 min at a pump flow rate of about 250 mL/min. Recirculation is enabled by closing off the filtrate outlet so that solution emerges from the retentate outlet only. After recirculation, the DEAE Column Buffer/KBr solution prepared previously is used to rinse out the ultrafiltration unit by stopping the pump and transferring the inlet tubing from the ultrafiltration unit to the reservoir containing the DEAE Column Buffer/KBr solution. The pump is then turned on and run until the total volume in the sample vessel is 655 - 660 mL, representing the final concentrated effluent solution, This solution is stored at 5 ± 3°C until KBr density gradient ultracentrifugation is performed.

(f) Density Gradient Ultracentrifugation

In preparing for the ultracentrifugation procedure. the concentrated effluent is allowed to warm to about 15°C. For each 650 mL of concentrated effluent, 90.8 g glycerol is added. After the glycerol addition, the sample's refractive index (RI) is measured. It should be 1.3890 ± 0.0030. If the sample's measured RI is below this value, KBr is added and dissolved until the required value is achieved. Should the measured RI exceed the required value, DEAE Column Buffer is added until the required value is obtained. After the required RI is obtained, the sample's final volume is measured. The sample is then dispensed into 40 mL polyallomer Quick-Seal tubes (Beckman catalog no. 342699) in 30.0 ± 0.5 mL aliquots. Once dispensed into the tubes, the samples are overlayed with a TNE/KBr solution, previously prepared as follows. 105.2 ± 0.1 g KBr is dissolved in 400 ± 5 mL TNE. The RI of this TNE/KBr solution should be 1.3620 ± 0.0002. If the measured value exceeds the required value, TNE is added until the correct RI is achieved. However, if the measured RI is less than the required value, KBr is added and dissolved until the appropriate value is obtained.

To insure that the overlay solution is delivered in a controlled manner, thus minimizing any disturbance of the sample-overlay interface, a peristaltic pump is used to initially deliver the overlay solution at a rate of 1 - 2 mL/min. For each tube, after the overlay solution has been applied to a height of more than 1 cm above the sample, the pump rate may be increased. After all the tubes have been filled, they are sealed and carefully placed in a Beckman 50.2 Ti rotor and loaded into a Beckman L8-55 ultracentrifuge (or equivalent). The samples are then spun at 45,000 RPM (245,000 x g) for 15.0 ± 0.5 hr at 15 ± 1°C.

While the samples are being centrifuged, the devices needed to remove the samples from the tubes can be readied. One or more peristaltic pumps (Pharmacia P1 or equivalent) are calibrated to deliver liquid at a flow rate of 4.00 ± 0.04 mL/min. Additionally, a Gilson Microfractionator fraction collector or equivalent is set up in conjunction with each calibrated peristaltic pump. The collector(s) are set to collect fractions at 0.5 min. intervals. 20 - 21 fractions will be collected from each tube. If only 20 fractions are to be collected per tube, the first 19 will contain 2.00 ± 0.02 mL each. If 21 fractions are to be collected per tube, the first 20 will contain 2.00 ± 0.02 mL each. In either case, the final fraction (fraction 20 or 21) may contain less than 2 mL.

Following completion of the ultracentrifugation, the sample-containing tubes are carefully extracted from the rotor and placed in a vertical position. During the sample removal/fractionation procedure, the samples can be maintained at 15 - 30°C. Once the pumps and fraction collectors have been set up, the top of each ultracentrifuge tube is carefully removed. 400 ± 20 µl is then carefully removed from each tube using a Pipetman P200 (Gilson) automatic pipeter or equivalent. This 400 µl should be withdrawn by placing the pipet tip just below the liquid's surface such that only solution from the top of the tube is removed. This initial 400 µl from each tube is placed in the last fraction container (20 or 21) for that particular tube. The remaining solution in each tube is removed by attaching a 100 µl Dade capillary pipette (or equivalent) to the inlet tubing of calibrated peristaltic pump from above. The pipette is carefully passed through the opening in the tube's top and then through the solution in the tube until it reaches the tube's bottom. The pump is then started. The fraction collector is turned on when liquid is about to emerge from the pump's outlet tubing. After all the fractions have been collected from a centrifuge tube, they should be stored at 5 ± 3°C. If 21 fractions were collected for a particular tube, fractions 20 and 21 are pooled. After each tube has been fractionated as described, all like numbered fractions from the various tubes are combined. Combined fractions 10 through 20 are then assayed in duplicate for the presence of HBsAg and for total protein content. In making dilutions for the HBsAg assay, assume the initial HBsAg concentration is 800 µg/mL. For both the HBsAg and total protein assays, aliquots removed from each fraction which are then used for making dilutions need not exceed 20 µl. The HBsAg assay is conducted using a commercially available immunoassay (Auszyme, Abbott Laboratories, Inc.). The total protein assay is conducted by the Bradford assay referred to above. After the HBsAg and total protein concentrations have been obtained for each of the combined fractions (10 - 20), the specific activity for each fraction can be calculated (HBsAg concentration [µg/mL] ÷ total protein concentration [µg/mL]). Those fractions having an HBsAg concentration of 130 μg/mL or more and a specific activity of greater than or equal to 0.2 are pooled and the volume measured. The total μg of HBsAg present in those fractions is then calculated.

(g) Gel Filtration Chromatography

The next step in the purification process involves gel filtration chromatography through three Sephacryl S-400 columns (each 13 cm x 50 cm) aligned in series which have previously been equilibrated with S-400 column Buffer (20 mM Tris-HCl, 135 mM NaCl, 0.21% [w/v] sodium deoxycholate, pH 8.2 - 8.5). The S-400 column should be operated in a cold room at 5 ± 3°C. The column's flow rate is controlled by attaching a peristaltic pump calibrated to deliver 80 ± 5 mL/hr to the column inlet tubing. Column effluent should be monitored spectrophotometrically at 275 - 285 nm and 1 AUFS and the measurements should be recorded by a chart recorder at a speed of 0.1 - 0.2 mm/min, with a range of 10 mv. S-400 Column Buffer is pumped through the column until a stable UV absorbance baseline is established, at which point the pump is turned off. An automated fraction collector is set up to collect effluent fractions of 13.0 ± 0.3 mL from the column.

Prior to loading the pooled HBsAg-containing density gradient fractions on to the column, a volume of 10% sodium deoxycholate (w/v) in WFI equal to 0.01 of the HBsAg sample's volume is added to the HBsAg-containing solution. The new volume of this solution is then recorded. This sodium deoxycholate/HBsAg-containing solution is then loaded on to the S-400 column at a rate of 80 ± 5 mL/min. Effluent monitoring and recording is also initiated. The first 250 ± 20 mL to come off the column is discarded. Fraction collection is then begun. 200 13 ± 3 mL fractions are then collected. The second UV-absorbing peak to be eluted from the S-400 column should be centered between fractions 100 - 120. That fraction representing the actual peak center is located. The 17 fractions to either side of that fraction are retained. 7 μl from each of these fractions is analyzed by SDS-PAGE with silver staining. Additionally, HBsAg and total protein content in each fraction is also assayed. For these assays, it can be assumed that the HBsAg concentration is 300 μg/mL and that the total protein concentration is 400 μg/mL. No more than 50 μl need be withdrawn from each these fractions for purposes of the assays. For purposes of economy, assays may be performed on only every other fraction. Interpolation can then be used to derive protein concentration values for those fractions not actually assayed. After the assays are complete, the specific activity (HBsAg concentration [μg/mL] ÷ total protein concentration [μg/mL]) of each fraction is calculated.

Those fractions having HBsAg concentrations in excess of 20 μg/mL and a specific activity of more than 0.4 are then pooled in a clean, tared container. After pooling the fractions, the container is again weighed and the net weight of the pooled material determined. An aliquot of this material is then removed and subjected to a Lowry protein assay. If the protein concentration as determined by the Lowry assay is less than 100 μg/mL, nothing more needs be done with the material, as this represents purified bulk HBsAg. However, should the protein concentration exceed 100 μg/mL, S-400 Column Buffer is added in an amount sufficient to adjust the protein concentration to 100 μg/mL, at which point this material represents purified bulk HBsAg. The purified bulk HBsAg is then stored at 5 ± 3°C until further use.

## EXAMPLE 5

### Alternative HBsAg Purification Method

The following example embodies the procedures used in a typical large-scale purification of HBsAg as produced in Example 2. Because the HBsAg so produced is to be used in a human vaccine formulation, all procedures described herein are performed in a manner to insure that the preparation is sterile and pyrogen free. Such procedures are common and known to those skilled in the art of pharmaceutical manufacturing. Unless otherwise indicated, at no time during the purification process should the temperature of the HBsAg-containing solution exceed 10°C.

(a) Cell Lysis

If the HBsAg(+) yeast cell culture is harvested by centrifugation, 23.0 ± 0.5 kilograms (kg) of freshly prepared or frozen cell paste is resuspended in 20 ± 0.5 L of resuspension buffer (50 mM Tris, 10 mM EDTA, pH 8.0) maintained at 5 ± 3°C. Alternatively, if the culture is harvested by filtration, 32.5 ± 0.5 kg of cell paste is dispersed in 10 L of resuspension buffer. Resuspension of the cell paste is conducted utilizing an overhead mixer. Once the cell paste is completely resuspended, the $OD_{600}$ of the solution is measured and the solution adjusted to an $OD_{600}$ of about 110 by adding additional resuspension buffer. The volume of the solution is then measured and glycerol added in an amount equal to 10% of the volume of the resuspended cell solution. Triton X-100® is then added to a final concentration of 0.5%. The pH of the resultant solution is adjusted to 7.9 ± 0.2 using 10 N sodium hydroxide (NaOH).

Cell lysis is accomplished by employing a Dyno Mill KD-5 (Glen Mills, Inc., Maywood, NJ) at a flow rate of 30 liters per hour (1/hr). The first 3,000 ± 25 mL collected is added back to the as yet unlysed portion of the cell suspension. Subsequent collection represents cell lysate. After the last of the cell suspension has entered the Dyno Mill, an additional 2.5 ± 0.5 L of lysis buffer is added to the reservoir. Lysate collection is stopped after the last of the lysis buffer enters the device.

(b) Clarification

The lysate is clarified by ultrafiltration/diafiltration using an Amicon MPD-100 filtration unit and a Millipore ProStak Module. The system contains 20 square feet of 0.65 μm membrane. In the filtration process, the lysate is concentrated to 50 ± 5% of its original volume. The concentrated filtrate is recovered and its temperature is not allowed to exceed 15°C. Cell wash buffer, comprised of 50 mM Tris-HCl, 10 mM EDTA, pH 8, is added to the retentate until the original volume of the lysate is restored. The concentration is repeated. Then the dilution and concentration are repeated two additional times. The filtrates from the four concentrations are combined and represent the clarified lysate.

(c) Adsorption

Following this clarification procedure, 40 grams (g) of the colloidal silicate Aerosil 380 is added per liter of clarified lysate over a 5 - 10 minute (min) period. An overhead mixer is used throughout the Aerosil addition to ensure thorough mixing. Mixing is continued an additional 8 - 16 hr at 5 ± 3°C. All mixing procedures employed in this purification process are to be at a speed sufficient to prevent settling of solution components. However, the mixing speed used should cause only minimal splashing and foaming of the solution.

HBsAg adsorbed to Aerosil 380 is separated from unadsorbed HBsAg by employing a Valley Foundry filter press comprised of seven plates and six frames, each measuring 18 inches by 18 inches, pre-coated with 900 g of Celite 535 filter-aid. 1,000 g of Celite 535 is mixed into the HBsAg/Aerosil 380 solution. This solution is then pumped through the filter press at a flow rate of 15 gallons per minute (gal/min), although the pressure in the press should never exceed 75 pounds per square inch. This separation can be carried out at room temperature (22 ± 5°C). The collected filtrate is discarded and pelleted material is completely resuspended at room temperature in TNE (20 mM Tris-HCl, 150 mM NaCl, 5 mM EDTA, pH 8) to a volume equal that of the clarified cell lysate (± 100 mL) prior to Aerosil 380 addition using a mixer. The solution is mixed for another 20 ± 10 min.

(d) Elution

The filter press is again coated with 900 g Celite 535 and the solution passed through the press at a flow rate of 15 gal/min. The filtrate is again discarded. The pellet is transferred to an appropriate container and resuspended in Aerosil 380 Elution Buffer (10 mM sodium borate, 1 mM EDTA, 0.25% [w/v] sodium deoxycholate, pH ~ 9.25) at room temperature in a volume equal that of the clarified lysate prior to the addition of the colloidal silicate ± 200 mL. After the pellet has been completely resuspended, the pH of the solution is adjusted to 9.25 ± 0.05 with 10 N NaOH. Mixing is then continued another 16-24 hr at room temperature. This solution is then passed through the Celite-coated filter press one additional time at a flow rate of 15 gal/min, this time retaining the filtrate, which contains eluted HBsAg. The pH of the solution is adjusted to 8.0 ± 0.1 using 2 N HCl and the solution's volume recorded. The Aerosil eluate is then adjusted to conditions of pH and ionic strength to match those of the DEAE cellulose to which this solution will be added. Conductivity of the solution, as measured at 22 + 2°C is adjusted to 14.3 ± 0.3 mmho/cm by adding 5 M NaCl. Record the solution's volume and store it at 5 ± 3°C.

(e) Ion Exchange Chromatography

16 kg of DEAE cellulose resin previously equilibrated in resin buffer comprised of 20 mM Tris-HCl, 5 mM EDTA, 110-120 mM NaCl, 0.02% (weight per volume) sodium metabisulfite, pH 8, is added per liter of pH adjusted, Aerosil 380 eluted HBsAg-containing solution. The solution is mixed at 5 ± 3°C until all the DEAE cellulose resin is evenly dispersed, from which point it is stirred an additional hour. The resultant slurry is then poured into two flatbed Buchner funnels (Bel Art H14621, 18″ inner diameter, 11.5″ depth, or equivalent), each connected to a peristaltic pump. Solution is pumped through the funnels at a rate of 5 L/min until the fluid level in the funnel reaches the top of the resin bed. Continue adding the DEAE cellulose/HBsAg-containing slurry until all of the initial solution has been loaded onto one of the two funnels and the solution level has fallen to the top of the resin beds. A volume of resin buffer equivalent to 30% of the volume of the HBsAg(+) eluate prior to the addition of DEAE cellulose is divided equally and poured into each of the two funnels. This solution is pumped at 5 L/min until the resin beds are dry. This solution is pooled with the earlier filtrate and the volumes measured.

The DEAE cellulose purified solution is then ultrafiltered and concentrated using an Amicon MPD-100 ultrafiltration system and one Romicon hollow fiber cartridge equilibrated by passing 25 ± 1 L of DEAE Column Buffer through the unit at a rate of 3 gal/min. Temperatures of the solutions so employed should not exceed 15°C. After loading the unit with the DEAE cellulose-purified material, the solution is pumped through the system at a rate of 7 gal/min with a filtrate rate of 1,000 ± 100 mL/min until the solution is concentrated to 60 ± 2 L. 60 ± 2 L of concentration diafiltration buffer (CDB; 10 mM sodium borate, 6 mM EDTA, 20 mM Tris-HCl, 111 mM NaCl, 0.02% sodium metabisulfite, pH 7.5), cooled to 4 ± 3°C, is then added to the retentate and again the solution is concentrated to 60 ± 2 L. This procedure is repeated one additional time and the concentration continued until the retentate volume is 20 ± 1 L. 420.0 ± 0.5 g of potassium bromide (KBr) is then added per liter of retentate. The solution is mixed at room temperature until all of the KBr is dissolved. Concentration of the solution is continued at a pump rate of 5 gal/min until the retentate volume reaches 10.0 ± 1 L. The concentrated solution is kept on ice to maintain a temperature of 10 ± 5°C. The pumping rate is reduced to about 3.0 gal/min. Concentration is continued until the retentate volume is 1.5 L, excluding hold-up volume. The solution is recirculated through the system for 5 ± 2 min but is concentrated no further. The retentate is pumped out of the system at a rate of 1 gal/min and the hold-up volume is combined with the retentate. This concentrated solution containing KBr is stored at 5 ± 3°C.

(f) Density Gradient Ultracentrifugation

Adjust the pH of the concentrated solution to 9.0 ± 0.1 using 10 N NaOH. Measure the total volume of the solution and add 0.14 g of glycerol per mL. Record the new volume after glycerol addition and readjust the pH to 9.0 ± 0.1 using 10 N NaOH. The refractive index (RI) of the solution should be 1.3890 ± 0.0030. If the RI of the solution is below this value, KBr is added and dissolved until the desired value is achieved. If the RI above 1.3890 ± 0.0030, DEAE resin buffer is added until the appropriate RI is obtained. If either KBR or DEAE resin buffer addition was required to achieve an RI value within the allowed range, measure and record the new volume of the solution. Store the solution at 5 ± 3°C until further use.

The overlay solution used in the KBr density gradient ultracentrifugation is prepared by adjusting the pH of 5,000 ± 50 mL TNE buffer to 9.0 ± 0.1 using 10 N NaOH. 1,315.0 ± 1.0 g KBr is then added to the solution and the RI measured. If the RI exceeds 1.3620 ± 0.0002, TNE buffer is added until the solution's RI falls within the desired range. If the RI is below the designated value, KBr is added and dissolved until the correct RI has been obtained. This overlay solution is used, in addition to overlaying the sample during the centrifugation, to prepare and bleed air from the density gradient system. After the K-3 ultracentrifuge rotor (Electronucleonics) has been degassed and spun at 10,000 revolutions per minute (rpm), the concentrated KBr-containing sample is pumped into the bottom of the rotor at a rate of 5 L/hr, displacing the overlay solution upward. After 500 ± 100 mL of the sample has been loaded, the pump rate is increased to 10 L/hr. During sample loading, the utmost care must be exercised to avoid the introduction of air bubbles into the rotor. Once loaded, the product-containing sample is accelerated to 35,000 RPM and spun for 8 ± 0.5hr. As the rotor is decelerated to 0 RPM, it contracts, forcing some of the sample out. This solution is collected and labelled the rotor contraction volume (RCV). After the rotor comes to a stop, the pump used to load the rotor is reversed and the rotor contents collected in 100 ± 20 mL fractions. These fractions are stored at 5 ± 3°C. Samples are withdrawn from each fraction and assayed for total protein content, HBsAg content, and a specific activity is calculated ([μg HBsAg/mL]/[μg total protein/mL]). Fractions containing more than about 130 μg/mL HBsAg with specific activities greater than about 0.2 are pooled and the pH of this solution adjusted to 8.2 ± 0.1 with 2 N HCl.

(g) Gel Filtration Chromatography

The pooled fraction sample is next subjected to gel filtration column chromatography. 0.21% weight per volume (w/v) sodium deoxycholate is added to the sample prior to gel filtration. Sephacryl S-400 resin (Pharmacia), equilibrated with 20 mM Tris-HCl, 135 mM Nacl, 0.21% (w/v) sodium deoxycholate, pH 8.5, is added to an appropriately sized column placed at 5 ± 3°C. Column flow rate, controlled by a peristaltic pump connected to the column inlet is 400 ± 40 mL/hour. Effluent from the column is continuously monitored at 275-280 nanometers (nm). After a stable effluent absorbance baseline has been established, the HBsAg-containing preparation is loaded onto the column. After 6.0 ± 0.5 L have been eluted, 75 fractions, each containing about 200 mL, are collected. During the chromatographic run, the second eluted peak of ultraviolet absorbing material should occur between fractions 55-70. The 17 fractions collected before and after that fraction representing the center of this absorbance peak are saved. Each of these fractions is then assayed for total protein and HBsAg content and a specific activity calculated. Fractions containing greater than 20 μg/mL HBsAg with a specific activity in excess of 0.4 are pooled. This material is stored at 5 ± 3°C and represents purified bulk HBsAg which may now be used in the preparation of a HBV vaccine.

EXAMPLE 6

HBsAg Particle Analysis

Quantitative lipid analysis of the highly purified HBsAg particle obtained in Example 4 was conducted as follows. Purified HBsAg, prepared as described in Example 2, was initially extensively dialysed against water for three days. After dialysis, the amount of ergosterol, a neutral lipid, present in the sample was determined. Standard solutions containing 0 - 80 μg ergosterol were prepared in 0.6 mL glacial acetic acid. Added to each of these solutions was 0.4 mL of ferric chloride reagent, prepared by combining 21 mL of concentrated sulfuric acid with 4 mL of a solution comprised of 500 mg ferric chloride in 20 mL water. The standard solutions were then mixed and allowed to stand 30 min before absorbances were measured at 550 nm to generate a standard curve. Triplicate samples of the purified, dialysed HBsAg preparation were then analyzed. 1 mL aliquots of the preparation were evaporated to dryness in a speed vac concentrator. Each pellet was then resuspended in 0.6 mL glacial acetic acid. After pellet dissolution, 0.4 mL of the ferric chloride reagent was added to each sample. The samples were thoroughly mixed and incubated at room temperature for 30 min. Just prior to the absorbance measurements at 550 nm, the samples were briefly centrifuged to pellet any particulates. The results showed an average of 0.063 μg ergosterol/μg starting protein. This result included both free and esterified ergosterol.

Samples of the preparation were then subjected to total lipid extraction to determine the types and relative amounts of neutral polar lipids present. This was done by combining 1 volume of purified, dialysed yeast derived HBsAg, 2.5 volumes of methanol, and 1.25 volumes of chloroform. This solution was vigorously mixed for 2 min. An additional 1.25 volumes of chloroform was then added and the solution mixed for an additional 30 sec. Finally, 1.25 volumes of water was added, followed by another 30 sec. of mixing. The phases were then allowed to resolve and the lower phase, containing chloroform and extracted lipids, was recovered.

Samples from this lipid-containing organic phase were then examined by thin layer chromatography on silica gel (linear high performance TLC plates utilizing a solvent comprised of hexane, ethyl ether, acetic acid, and methanol in a 60:40:1:1 ratio, respectively. After sample migration was complete, the plates were dried. Neutral lipids were then visualized by spraying the plates with 10 N sulfuric acid. The plates were then scanned using a Shimadzu flying spot densitometer in the reflectance mode. The results obtained revealed that ergosterol, triglycerides, and ergosterol esters were present in a 15.8:10.1:45.3 ratio, respectively. Thus, the neutral lipids in the yeast derived HBsAg preparation are comprised of 22% ergosterol, 14% triglycerides, and 64% ergosterol esters.

Each μg of starting protein is known to be associated with 0.063 μg ergosterol. This quantity of ergosterol is actually comprised of 0.014 μg ergosterol and 0.049 μg of ergosterol in the form of ergosterol ester. 0.049 μg of ergosterol in the form of ergosterol ester represents 0.085 μg of ergosterol ester. Thus, there is 0.099 μg of total sterol (ergosterol plus ergosterol ester) per μg of starting protein. Ergosterol and ergosterol esters account for 84% of the neutral lipid found in the yeast derived HBsAg samples. 0.016 μg of triglycerides are also present per mg of protein. Therefore, the analyzed HBsAg preparation contains 0.115 μg neutral lipid/μg protein.

Polar lipids, such as phosphatidyl choline, phosphatidyl ethanolamine, phosphatidyl serine, and phosphatidyl inositol, are detected by three dimensional thin layer chromatography on silica gel (linear high performance) TLC plates. The first dimension is chromatographed using a solvent comprised of chloroform, methanol, and ammonium hydroxide in a 65:25:4 ratio, respectively. The solvent is allowed to run the length of the plate before the plate is removed from the solvent and allowed to dry. The second dimension is performed in the same direction utilizing the same solvent. After the solvent has migrated the length of the plate, the plate is again removed and allowed to dry thoroughly. Chromatography in the third dimension is performed by placing the now dried plate at a right angle to its original migration direction in a solvent comprised of chloroform, acetone, methanol, acetic acid, and water in a 30:40:10:10:5 ratio, respectively. After the third dimension migration is completed, the plate is again removed from the solvent and dried. The various phospholipids present are visualized by charring with phospray or 10 N sulfuric acid. Analysis of the yeast derived HBsAg preparation described above showed very little polar lipid to be present. The only species detected was phosphatidyl choline. Significant quantities of deoxycholate were detected in the purified, dialysed HBsAg samples.

In contrast, samples of human serum derived HBsAg, identically extracted, run on the same plate, and containing equivalent amounts of protein as the purified, dialysed yeast derived samples, revealed a significant level of phosphatidyl choline and no deoxycholate.

Because of the disparity in the phospholipid levels seen in the HBsAg preparation generated as presently described and in human serum derived HBsAg preparations, efforts to determine the amount of phospholipids present in the various samples were undertaken. A standard containing 20 μg/mL inorganic phosphate was purchased. Duplicate aliquots containing 0 - 1.6 μg phosphorus were dispensed into glass tubes. 0.3 mL of 10 N sulfuric acid was added to each tube. The standards were then heated 3 hr at 150°C to oxidize any protein or organic compound present. Such compounds, if present, turn black during this treatment. Several drops of $H_2O_2$ were then added to each standard sample and heating was continued an additional hr, totally eliminating any color from the tubes. During this incubation, all water was boiled off and all samples were reduced to the same volume in sulfuric acid.

After the samples were removed from the oven and cooled, 0.65 mL water was added to each tube, followed by 200 μl of 5% ammonium molybdate and 50 μl of Fiske-Subbarow reagent. The samples were then placed in a boiling water bath for 7 min, allowing stable color to develop. The samples' absorbances were then read at 830 nm to develop a standard curve. HBsAg purified from human serum contained 9.6 x $10^{-3}$μg phosphorus/μg HBsAg protein, which corresponds to 0.23 μg phospholipid/μg protein [phospholipid molecular weight (750 g/mol) divided by the molecular weight of phosphorus (31 g/mol), multiplied by 9.6 x $10^{-3}$ μg phosphorus/μg HBsAg protein], while yeast derived HBsAg, purified as described in Example 2 and then dialysed, contained 4.4 x $10^{-3}$ μg phosphorus/μg protein, or 0.10 μg phospholipid/μg protein. The result obtained for the purified, dialysed yeast derived material conflicted with the results obtained in the polar lipid TLC analysis because 0.10 mg phospholipid/mg protein would have been readily detectable under the TLC condition employed. These results suggested the phosphorus present in the phosphate determination must have come from a source other than phospholipids.

To determine how much of the phosphorus present was due to phospholipids, a sample of the purified, dialysed yeast derived HBsAg was again subjected to lipid extraction. Phosphorus quantitation was then performed on both the lipid-containing organic phase and the aqueous phase. These results revealed that 3.05 x $10^{-3}$μg phosphorus/μg protein was present in the aqueous phase, while 5.88 x $10^{-4}$ μg phosphorus/μg protein, or 0.014 μg phospholipid/μg protein was present in the organic phase. These results demonstrate that only about 16% of the phosphorus present in the purified yeast-derived HBsAg preparation is due to phospholipids, and that greater than 90% of the phospholipids had been extracted and replaced by deoxycholate. The remaining phosphorus in both the human serum derived and purified yeast derived preparations may be protein associated, as is indicated by the inability of repeated chloroform/methanol (2:1), water, acetone, ethanol, or methanol washings to remove the remaining phosphorus.

The above procedures enabled the determination of the composition of the purified, dialysed yeast-derived HBsAg preparation. The lipid to protein ratio was found to be 0.120 to 1.00. The neutral lipid to polar lipid ratio was found to be 0.115 to 0.014. The neutral lipids were found to be comprised of 0.014 μg ergosterol/μg protein, 0.085 μg esterified ergosterol/μg protein, and 0.016 μg triglycerides/μg protein. The polar lipids were comprised mainly of 0.014 μg phosphatidyl choline/μg protein. Thus, yeast derived HBsAg, when prepared as described above, is comprised of approximately 11% lipid and 89% protein, excluding the presence of significant quantities of deoxycholate. The amount of deoxycholate was determined as follows. Total lipid was extracted into a chloroform/methanol phase; 1.9 mg of deoxycholate per 8.4 mg of protein was recovered. By total phosphate analysis, combined with neutral lipid analysis, only 0.166 mg was lipid. The remainder (~92%) was deoxycholate, as verified by its mobility on TLC plates.

## EXAMPLE 7

### Adjuvanting Procedures

The following adjuvanting procedure is used to prepare HBV vaccine formulations incorporating HBsAg as produced and purified above in Examples 2, 3, and 4. This adjuvanting protocol may be used for preparations containing from 5-200 μg/mL of purified bulk HBsAg. All of the described procedures are to be carried out aseptically.

Working back from the desired final dosage of HBsAg, one calculates the ratio (A) of 5% alum (aluminium hydroxide) solution which is to be added to the purified HBsAg-containing bulk material according to the mathematical equation: A = 1/C x (0.178), where C is the desired final concentration of HBsAg expressed in mg/mL. The amount of 5% alum solution to be added is calculated as the product of the ratio (A) and the total number of milligrams of HBsAg to be adjuvanted. The calculated amount of 5% alum solution is then divided equally among an appropriate number of pre-weighed centrifuge bottles sufficient in size to hold the adjuvanted purified bulk material. 0.25 N NaOH is then added to each centrifuge bottle in an amount equal to the product of the volume of the 5% alum solution added to that bottle and 1.136. The resultant solution is mixed thoroughly by gently swirling the centrifuge bottles. The appropriate quantity of purified bulk material is then added to each bottle. The bottles are then capped, sealed, and placed on a rotary shaker at 150-200 RPM at room temperature for 2 hours ± 10 minutes. The bottles are then removed from the shaker and spun at 1,000 x g for approximately 15 ± 1 min. at 20°C. The supernatants from this centrifugation are gently decanted so as to avoid disturbing the pellets. PBS (phosphate buffered saline; 1.5 mM $Na_2HPO_4$, 2.2 mM $NaH_2PO_4$, 150 mM NaCl, pH 6.5) is added to an amount equal to 20% of the total volume of the centrifuge bottles. Each bottle is sealed and swirled gently to resuspend the pellets. Upon resuspension of the pellets, the bottles are reopened and PBS is added to each in an amount sufficient to fill the bottles to 70% of their maximum capacity. The bottles are then centrifuged once more at 1,000 x g for 15 ± 1 min. Again the supernatants are decanted carefully. The weights of the pellets, representing the total amount of adjuvanted HBsAg, are then determined.

An adjuvanted HBsAg preparation containing a specified final concentration of HBsAg is then produced by adding and gently resuspending the pellets in the amount of PBS necessary to produce the desired concentration of antigen. The resuspended pellets are then pooled in an appropriate container. This adjuvanted HBsAg preparation is then stored at 2 - 8°C until it is vialed and packaged for distribution.

Because the amount of antigen needed to generate protective immunity to Hepatitis B infection varies depending upon the age of the patient to be vaccinated, vaccine formulations having HBsAg present in different concentrations may be necessary. To produce formulations containing different concentrations of HBsAg, dilutions of the adjuvanted HBsAg-containing Hepatitis B vaccine formulation as described above can be produced by adding an appropriate amount of PBS, containing an appropriate amount of 5% alum, to a specific volume of the previously adjuvanted vaccine material. Hepatitis B vaccines, manufactured by the above procedures, are now suitable for human administration.

In an alternative adjuvanting procedure, appropriate solutions are initially prepared. S-400 Column Buffer (20 mM Tris-HCl, 135 mM NaCl, 0.21% (w/v) sodium deoxycholate, pH 8.5; same buffer as used in gel filtration chromatography in Examples 3 and 4) and PBS are filtered through a 47 mm, 142 mm, or 293 mm Pall Nylon 66™ filter (Pall Corp.) having a 0.20 μm pore size. The filtered solutions are then stored at 2 - 8°C until needed. Additionally, appropriate volumes of 0.25 N NaOH and 5% Alum (aluminium phosphate) are filtered using a 0.2 μm nylon filter for chemical clarification for non-aseptic purposes.

Following filtration of the component solutions, the desired volume (actually measured in terms of solution weight) of concentrated aluminium hydroxide (AlOH) gel is prepared. In an appropriately sized container, for each kg of AlOH desired, 1758 g of 5% Alum is added, followed by the addition of 1998 g 0.25 N NaOH. This solution is then stirred 15 ± 2 min at room temperature. After 15 min has elapsed, 625 mL aliquots of this solution are placed in 1 L centrifuge bottles (agitation of the solution is continued as the bottles are loaded). The samples are then spun at 1,800 RPM in a Beckman J6B rotor. Supernatants are then discarded and each AlOH pellet is resuspended with 70 g PBS. The resuspended pellets are then transferred and combined in 1 L glass sterilization bottles (6 resuspended pellets per bottle). The centrifuge bottles are then rinsed with 10 g PBS and these rinse solutions are then pooled with the previously resuspended pellets. Each sterilization bottle is then weighed and PBS is added until the weight of the contents reaches 1 kg. These solutions represent concentrated AlOH gel, which are then steam sterilized in a validated cycle, such as 3 hours at 124 ± 1°C. Following sterilization, samples from one bottle are subjected to aluminium analysis by atomic absorbtion to determine the concentration of AlOH in terms of parts per million (ppm). The remaining AlOH gel is stored at 2 ± 8°C until needed.

The sterilized AlOH gel is then used to adjuvant sterile, filter purified HBsAg as prepared in Example _. Initially, calculations are made to achieve an intermediate HBsAg concentration of 0.050 mg/mL. The following equation is used to make this calculation:

a) starting HBsAg weight (g) x starting HBsAg concentration (mg/mL) ÷ 0.050 mg/mL (intermediate HBsAg concentration) = intermediate HBsAg weight (g).

This calculated intermediate weight is then used to ascertain the final weight of the adjuvanted HBsAg formulation by employing the following equation:

b) intermediate weight (g) x 0.050 mg/mL (intermediate concentration) ÷ desired final HBsAg concentration (mg/mL) = final solution weight (g).

The amount of concentrated AlOH gel to add is calculated as follows:

c) desired final aluminium concentration (ppm) ÷ aluminium concentration (ppm) in concentrated AlOH gel x final solution weight = amount of concentrated AlOH gel to add.

A bottle containing concentrated AlOH gel is stirred for 10 min. The amount of S-400 Column Buffer needed for adjuvanting is calculated as follows:

d) intermediate weight (g) - HBsAg starting weight (g) = amount of S-400 Column Buffer (g) needed.

The amount of PBS needed is calculated as follows:

e) final solution weight (g) containing the desired final concentration of HBsAg (mg/mL) - intermediate weight (step a) - concentrated AlOH gel to add (step c) = PBS needed (g).

The calculated amount of S-400 Column Buffer is then placed into the previously weighed final receiving vessel. After re-taring, the amount of PBS calculated in (step e) is then added to the final receiver. After re-taring, the amount of stirred, concentrated AlOH gel needed (step c) is added. Finally, after re-taring, the amount (weight) of sterile, filter purified HBsAg to be adjuvanted is added. The resultant solution is stirred at a slow to moderate speed for 15 min. The final weight of this solution should correspond to that calculated in step b. This bulk adjuvanted HBsAg formulation, having a final HBsAg concentration appropriate for vaccine administration, is now ready to be vialed and included in a vaccine kit.

## EXAMPLE 8

### Vaccine Administration

HBV vaccines containing HBsAg as the immunogenic molecule typically are delivered by intramuscular (im) injection in humans. The deltoid muscle is the preferred site for im injection in children and adults, while in neonates and infants, the preferred injection site is the anterolateral thigh. Data suggest that HBV vaccine injections in the buttocks frequently are given into fatty tissue instead of into muscle, resulting in lower seroconversion rates than would otherwise occur and thus reducing the vaccine's effectiveness in preventing Hepatitis B infection. However, as mentioned previously, in persons at risk of hemorrhage following im injection, the HBsAg-derived HBV vaccine should be administered subcutaneously. The vaccine formulation as produced herein should not be injected into or near blood vessels or nerves.

The immunization regimen consists of three vaccine doses given as follows: Dose 1 is given on some elected date; Dose 2 is given 30 days after the first dose; and Dose 3 is given 180 days after the initial vaccine injection. The duration of protection from Hepatitis B infection following immunization with the HBV vaccine formulation as manufactured herein has not been determined. Studies with other HBsAg-derived HBV vaccines have shown that antibodies to HBV may fall below 10 mIU/mL (milli-International units), the level of antibodies considered necessary to provide protection against HBV infection in clinical trials [Stevens et al., (1987) *JAMA,* vol. 257, p. 2612], over three to four years following immunization. In one study, 71% of adults who initially responded to the vaccine with protective antibodies retained levels of greater than or equal to 10 mIU/mL after 4.5 years post-immunization. In addition, among those who no longer exhibited detectable levels of protective antibodies, protection against chronic hepatitis appeared to persist (*Physicians' Desk Reference,* supra). For individuals likely to be exposed to Hepatitis B infection, revaccination with a single booster injection is recommended when antibodies to HBV fall below 10 mIU/mL.

The dosage of HBsAg delivered to an individual being immunized is determined by age and condition of the patient. An immunologically effective amount of HBsAg is that amount of HBsAg which must be administered to a patient to produce the desired immunological effect, i.e. to protectively immunize the patient against future HBV infection. As little as 2.5 µg of HBsAg per injection can be administered to small children (under age 11), while up to 40 µg of HBsAg per dose can be given to adult hemodialysis patients. Typical adult dosages incorporate about 10 - 20 0µg HBsAg per immunization, while child dosages range from about 2.5 µg HBsAg to 10 µg of HBsAg for neonates whose mothers are known to be HBsAg-positive.

## EXAMPLE 9

### Comparison of Hepatitis B Vaccines

Several Hepatitis vaccines, prepared either from the plasma of Hepatitis B afflicted individuals or by recombinant techniques, have been compared in China for their ability to protect infants born to mothers who are HBsAg and HBeAg-positive from HBV infection. Included among the vaccines tested are those currently available in the United States, Recombivax HB®, Engerix-B®, and Heptavax-B®. The infants studied were immunized according to the vaccination regimen as detailed in Example 8 of the present invention. The first dose was administered immediately after birth or at one month of age. Subsequent immunizations were administered one month later and six months after the initial injection. No HBIG was administered to any of the infants during the course of this study. Development of protective antibodies to HBV was traced over a 9 month period. See Table 5 for the results of the study.

TABLE 5

| VACCINE | # of infants | μg HBsAg | # of infants after 8-9 mo. HBsAg (+) | % of infants protected | # of infants having HBV antibodies after 8-9 mo. | GMT (mIU/ml) | mIU range |
|---|---|---|---|---|---|---|---|
| AMGEN (yeast) | 100 | 10 | 13 | 85.6 | 84 | 884.44 | 55.0-3388 |
| MERCK (yeast) | 96 | 5 | 19 | 78 | 58 | 264.7 | 1.8-2138 |
| MERCK (plasma) | 92 | 10 | 24 | 71 | 43 | 281.7 | 8.7-4168 |
| SMITHKLINE (yeast) | 72 | 20 | 17 | 73.3 | 54 | 164.46 | 4.5-267 |
| CHINA 853-1 (plasma) | 29 | 15 | 12 | 54.1 | 15 | 144.1 | 7.4-977 |
| CHINA 853-2 (plasma) | 78 | 15 | 32 | 54.4 | 44 | 81.6 | 5.8-1753 |
| CHINA 831 (plasma) | 42 | 40 | 5 | 86.6 | 38 | 359.6 | N/A |
| CONNAUGHT (C127) | 50 | 20 | 48 | 72.2 | 38 | 149.7* | |

* = 7 mo.

As can be seen from Table 5, when plasma derived Hepatitis B vaccines is utilized, 20 - 30 μg of HBsAg per injection is required to significantly prevent transmission of HBV from mother to infant. Injections containing only 10 μg of HBsAg generally failed to block HBV infection over a three year period. On the other hand, recombinantly derived vaccines generated antibodies to HBV with geometric mean titers (GMTs) higher than those elicited by the plasma derived vaccines containing equal amounts of HBsAg. Importantly, a 70 - 80% infection blocking rate was obtained when 5 - 10 μg of recombinantly derived HBsAg was present in the vaccine formulations. The Hepatitis B vaccine formulation taught by the present invention produced much higher antibody titers to HBV in infants than any other vaccine tested.

While the present invention has been described in terms of preferred embodiments, it is understood that variations and modifications will occur to those skilled in the art in light of the above description.
Therefore, it is intended that the appended claims cover all such variations which come within the scope of the invention as claimed.

The features disclosed in the foregoing description, in the following claims and/or in the accompanying drawings may, both separately and in any combination thereof, be material for realising the invention born diverse forms thereof.

## Claims

1. A Hepatitis B vaccine formulation suitable for administration to mammalian species comprising:
   a) an immunologically effective amount of HBsAg; and
   b) a bile acid salt.

2. A vaccine formulation according to Claim 1 wherein the bile acid salt comprises at least about 10% by weight of the HBsAg particle.

3. A vaccine formulation according to Claim 1 wherein the amount of the bile acid salt ranges from about 10% to about 30% by weight of the HBsAg particle.

4. A vaccine formulation according to Claim 3 wherein the bile acid salt represents about 20% of the HBsAg particle by weight.

5. A vaccine formulation according to Claim 1 which additionally includes from about 2% to about 12% lipid by weight.

6. A vaccine formulation according to Claim 5 wherein the lipid is of yeast origin.

7. A vaccine formulation according to Claim 1 wherein the bile acid salt is deoxycholate.

8. A vaccine formulation according to Claim 7 wherein the deoxycholate is sodium deoxycholate.

9. A vaccine formulation according to Claim 1 wherein the HBsAg is of recombinant origin.

10. A vaccine formulation according to Claim 9 wherein the HBsAg is produced as the result of recombinant yeast host cell expression.

11. A vaccine formulation according to Claim 10 wherein the yeast host cell is *S. cerevisiae.*

12. A Hepatitis B vaccine formulation suitable for administration to mammalian species comprising:
    a) recombinantly derived HBsAg produced in the yeast *S. cerevisiae;*
    b) sodium deoxycholate, which comprises about 20% of the HBsAg particle by weight; and
    c) yeast-derived lipid, which comprises about 2% to about 12% of the HBsAg particle by weight.

13. A method for developing a protective immunological response in a mammalian species susceptible to hepatitis B viral infection comprising vaccination of the mammal with a vaccine according to Claim 1.

14. A method according to Claim 13 wherein the mammal is a human.

**15.** A method according to Claim 13 wherein the the bile acid salt ranges from about 10% to about 30% by weight of the HBsAg particle.

**16.** A method according to Claim 13 wherein the HBsAg particle additionally includes from about 2% to 12% yeast-derived lipid by weight.

**17.** A method according to Claim 13 wherein the bile acid salt is deoxycholate.

**18.** A method according to Claim 17 wherein the deoxycholate is sodium deoxycholate.

**19.** A method according to Claim 13 wherein the HBsAg is of recombinant origin.

**20.** A method according to Claim 19 wherein the HBsAg is produced as the result of recombinant yeast host cell expression.

**21.** A method according to Claim 20 wherein the yeast host cell was *S. cerevisiae.*

**22.** A method for purifying HBsAg suitable for use in a vaccine formulation as in Claim 1 comprising the steps of:
a) lysing of the host cells in which HBsAg was expressed;
b) clarifying the HBsAg-containing lysate from step (a);
c) adsorbing the HBsAg in the HBsAg-containing lysate from step (b) to a colloidal silicate;
d) eluting adsorbed HBsAg from the colloidal silicate in step (c);
e) subjecting the eluted HBsAg from step (d) to ion exchange chromatography;
f) subjecting the ion exchange-purified HBsAg from step (e) to density gradient ultracentrifugation;
g) subjecting the ultracentrifuge-purified HBsAg from step (f) to gel filtration chromatography; and
h) recovering highly purified bulk HBsAg from the gel filtration in step (g).

**23.** A method according to Claim 22 wherein the colloidal silicate is Aerosil 380.

**24.** A method according to Claim 22 wherein the gel chromatography employs a bile acid salt.

**25.** A method according to Claim 24 wherein the bile acid salt is deoxycholate.

**26.** A method according to Claim 25 wherein the bile acid salt is sodium deoxycholate.

**27.** A method according to claim 22 further comprising the the step of adjuvanting the purified bulk HBsAg.

**28.** A method according to Claim 27 wherein the adjuvant used is selected from the group consisting of aluminium hydroxide and aluminium phosphate.

**29.** A method according to Claim 28 wherein the adjuvant used is aluminium hydroxide.

**30.** A method for carbon-limited cultivation of a yeast host strain containing an exogenous DNA sequence comprising the steps of:
a) initially growing the culture at a temperature between 20°C and 30°C at a pH of 3.5 - 6.0; and
b) after the culture reaches an appropriate cell density, reducing the culture temperature to about 15°C ± 3°C.

**31.** A method according to Claim 30 wherein the initial culture temperature is 25°C ± 1°C.

**32.** A method according to Claim 30 where the pH in step(a) is 4.5 ± 0.2.

**33.** A method according to Claim 30 wherein the pH in step(a) is 4.5 ± 0.2 and after reducing the culture temperature to 15°C ± 3°C, the culture pH is adjusted to 5.5 ± 0.2.

**34.** A method according to Claim 30 wherein the yeast host strain is *S. cerevisiae.*

**35.** A method according to Claim 30 wherein the exogenous DNA fragment comprises a recombinant plasmid directing the expression of HBsAg.

**36.** A method according to Claim 35 wherein the recombinant plasmid is pGPD-1(HBS).

# FIGURE 1

```
AAGCTTGGCT GCAGGTCGAG TTTATCATTA TCAATACTGC CATTTCAAAG   50
AATACGTAAA TAATTAATAG TAGTGATTTT CCTAACTTTA TTTAGTCAAA  100
AAATTAGCCT TTTAATTCTG CTGTAACCCG TACATGCCCA AAATAGGGGG  150
CGGGTTACAC AGAATATATA ACATCGTAGG TGTCTGGGTG AACAGTTTAT  200
TCCTGGCATC CACTAAATAT AATGGAGCCC GCTTTTTAAG CTGGCATCCA  250
GAAAAAAAAA GAATCCCAGC ACCAAAATAT TGTTTTCTTC ACCAACCATC  300
AGTTCATAGG TCCATTCTCT TAGCGCAACT ACAGAGAACA GGGGCACAAA  350
CAGGCAAAAA ACGGGCACAA CCTCAATGGA GTGATGCAAC CTGCCTGGAG  400
TAAATGATGA CACAAGGCAA TTGACCCACG CATGTATCTA TCTCATTTTC  450
TTACACCTTC TATTACCTTC TGCTCTCTCT GATTTGGAAA AAGCTGAAAA  500
AAAAGGTTGA AACCAGTTCC CTGAAATTAT TCCCCTACTT GACTAATAAG  550
TATATAAAGA CGGTAGGTAT TGATTGTAAT TCTGTAAATC TATTTCTTAA  600
ACTTCTTAAA TTCTACTTTT ATAGTTAGTC TTTTTTTTAG TTTTAAAACA  650
CCAAGAACTT AGTTTCGACG GATCCCGAAT AAACACACAT AAATAAACAA  701

ATG GAA AAC ATT ACT TCT GGT TTC TTG GGT CCA TTG TTG GTT  743
Met Glu Asn Phe Thr Ser Gly Phe Leu Gly Pro Leu Leu Val

TTG CAA GCT GGT TTC TTC TTG TTG ACT AGA ATC TTG ACT ATT  785
Leu His Ala Gly Phe Phe Leu Leu Thr Arg Ile Leu Thr Ile

CCA CAA AGT CTA GAC TCG TGG TGG ACT TCT CTC AAT TTT CTA  827
Pro His Ser Leu Asp Ser Trp Trp Thr Ser Leu Asn Phe Leu

GGG GGA TCA CCC GTG TGT CTT GGC CAA AAT TCG CAG TCC CCA  869
Gly Gly Ser Pro Val Cys Leu Gly Gln Asn Ser Gln Ser Pro

ACC TCC AAT CAC TCA CCA ACC TCC TGT CCT CCA ATT TGT CCT  911
Thr Ser Asn His Ser Pro Thr Ser Cys Pro Pro Ile Cys Pro

GGT TAT CGC TGG ATG TGT CTG CGG CGT TTT ATC ATA TTC CTC  953
Gly Tyr Arg Trp Met Cys Leu Arg Arg Phe Ile Ile Phe Leu
```

FIGURE 1A

```
TTC ATC CTG CTG CTA TGC CTC ATC TTC TTA TTG GTT CTT CTG  995
Phe Ile Leu Leu Leu Cys Leu Ile Phe Leu Leu Val Leu Leu

GAT TAT CAA GGT ATG TTG CCC GTT TGT CCT CTA ATT CCA GGA  1037
Asp Tyr Gln Gly Met Leu Pro Val Cys Pro Leu Ile Pro Gly

TCA ACA ACA ACC AGT ACG GGA CCA TGC AAA ACC TGC ACG ACT  1079
Ser Thr Thr Thr Ser Thr Gly Pro Cys Lys Thr Cys Thr Thr

CCT GCT CAA GGC AAC TCT ATG TTT CCC TCA TGT TGC TGT ACA  1121
Pro Ala Gln Gly Asn Ser Met Phe Pro Ser Cys Cys Cys Thr

AAA CCT ACG GAT GGA AAT TGC ACC TGT ATT CCC ATC CCA TCG  1163
Lys Pro Thr Asp Gly Asn Cys Thr Cys Ile Pro Ile Pro Ser

TCC TGG GCT TTC GCA AAA TAC CTA TGG GAG TGG GCC TCA GTC  1205
Ser Trp Ala Phe Ala Lys Tyr Leu Trp Glu Trp Ala Ser Val

CGT TTC TCT TGG CTC AGT TTA CTA GTG CCA TTT GTT CAG TGG  1247
Arg Phe Ser Trp Leu Ser Leu Leu Val Pro Phe Val Gln Trp

TTC GTA GGG CTT TCC CCC ACT GTT TGG CTT TCA GCT ATA TGG  1289
Phe Val Gly Leu Ser Pro Thr Val Trp Leu Ser Ala Ile Trp

ATG ATG TGG TAT TGG GGG CCA AGT CTG TAC AGC ATC GTG AGT  1331
Met Met Trp Tyr Trp Gly Pro Ser Leu Tyr Ser Ile Val Ser

CCC TTT ATA CCG CTG TTA CCA ATT TTC TTT TGT CTC TGG GTA  1373
Pro Phe Ile Pro Leu Leu Pro Ile Phe Phe Cys Leu Trp Val

TAC ATT TAA                                              1382
Tyr Ile
```

FIGURE 2

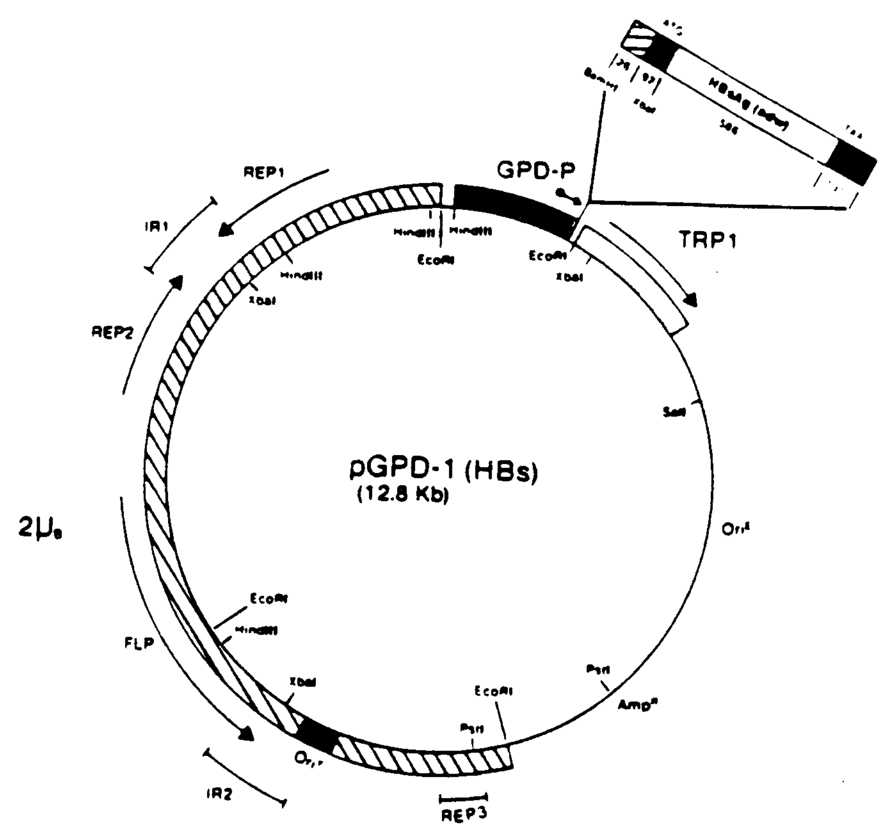
GPD-P
TRP1
REP1
IR1
REP2
HindIII
EcoRI
XbaI
SalI
pGPD-1 (HBs)
(12.8 Kb)
2μ
FLP
PstI
AmpR
IR2
REP3

SEQUENCE LISTING

(1) GENERAL INFORMATION:

(i) APPLICANT: Langley, K.E.
Bitter, G.A.
Sachdev, R.K.
Fieschko, J.C.

(ii) TITLE OF INVENTION: A Hepatitis B Vaccine Formulation Incorporating a Bile Acid Salt

(iii) NUMBER OF SEQUENCES: 1

(iv) CORRESPONDENCE ADDRESS:

(A) ADDRESSEE: Amgen Inc.

(B) STREET: Amgen Center
1840 Dehavilland Drive

(C) CITY: Thousand Oaks

(D) STATE: California

(E) COUNTRY: USA

(F) ZIP: 91320-1789

(v) COMPUTER READABLE FORM:

(A) MEDIUM TYPE: Diskette, 3.5 in., DS, 1.4 MB

(B) COMPUTER: Apple Macintosh

(C) OPERATING SYSTEM: Macintosh OS 6.0.4

(D) SOFTWARE: Microsoft Word Version 4.0b

(vi) CURRENT APPLICATION DATA:

(A) APPLICATION NUMBER:

(B) FILING DATE: 20-SEPT-1991

(C) CLASSIFICATION:

(2) INFORMATION FOR SEQ ID NO:1:

(i) SEQUENCE CHARACTERISTICS:

(A) LENGTH: 1382 base pairs

(B) TYPE: nucleic acid

(C) STRANDEDNESS: double stranded

(D) TOPOLOGY: circular

(ii) other nucleic acid

(xi) SEQUENCE DESCRIPTION: SEQ ID NO:1:

```
AAGCTTGGCT GCAGGTCGAG TTTATCATTA TCAATACTGC CATTTCAAAG   50
AATACGTAAA TAATTAATAG TAGTGATTTT CCTAACTTTA TTTAGTCAAA  100
AAATTAGCCT TTTAATTCTG CTGTAACCCG TACATGCCCA AAATAGGGGG  150
CGGGTTACAC AGAATATATA ACATCGTAGG TGTCTGGGTG AACAGTTTAT  200
TCCTGGCATC CACTAAATAT AATGGAGCCC GCTTTTTAAG CTGGCATCCA  250
GAAAAAAAAA GAATCCCAGC ACCAAAATAT TGTTTTCTTC ACCAACCATC  300
AGTTCATAGG TCCATTCTCT TAGCGCAACT ACAGAGAACA GGGGCACAAA  350
CAGGCAAAAA ACGGGCACAA CCTCAATGGA GTGATGCAAC CTGCCTGGAG  400
TAAATGATGA CACAAGGCAA TTGACCCACG CATGTATCTA TCTCATTTTC  450
TTACACCTTC TATTACCTTC TGCTCTCTCT GATTTGGAAA AAGCTGAAAA  500
AAAAGGTTGA AACCAGTTCC CTGAAATTAT TCCCCTACTT GACTAATAAG  550
TATATAAAGA CGGTAGGTAT TGATTGTAAT TCTGTAAATC TATTTCTTAA  600
ACTTCTTAAA TTCTACTTTT ATAGTTAGTC TTTTTTTTAG TTTTAAAACA  650
CCAAGAACTT AGTTTCGACG GATCCCGAAT AAACACACAT AAATAAACAAA 701
```

```
ATG GAA AAC ATT ACT TCT GGT TTC TTG GGT CCA TTG TTG GTT    743
Met Glu Asn Phe Thr Ser Gly Phe Leu Gly Pro Leu Leu Val

TTG CAA GCT GGT TTC TTC TTG TTG ACT AGA ATC TTG ACT ATT    785
Leu His Ala Gly Phe Phe Leu Leu Thr Arg Ile Leu Thr Ile

CCA CAA AGT CTA GAC TCG TGG TGG ACT TCT CTC AAT TTT CTA    827
Pro His Ser Leu Asp Ser Trp Trp Thr Ser Leu Asn Phe Leu

GGG GGA TCA CCC GTG TGT CTT GGC CAA AAT TCG CAG TCC CCA    869
Gly Gly Ser Pro Val Cys Leu Gly Gln Asn Ser Gln Ser Pro

ACC TCC AAT CAC TCA CCA ACC TCC TGT CCT CCA ATT TGT CCT    911
Thr Ser Asn His Ser Pro Thr Ser Cys Pro Pro Ile Cys Pro

GGT TAT CGC TGG ATG TGT CTG CGG CGT TTT ATC ATA TTC CTC    953
Gly Tyr Arg Trp Met Cys Leu Arg Arg Phe Ile Ile Phe Leu

TTC ATC CTG CTG CTA TGC CTC ATC TTC TTA TTG GTT CTT CTG    995
Phe Ile Leu Leu Leu Cys Leu Ile Phe Leu Leu Val Leu Leu

GAT TAT CAA GGT ATG TTG CCC GTT TGT CCT CTA ATT CCA GGA   1037
Asp Tyr Gln Gly Met Leu Pro Val Cys Pro Leu Ile Pro Gly

TCA ACA ACA ACC AGT ACG GGA CCA TGC AAA ACC TGC ACG ACT   1079
Ser Thr Thr Thr Ser Thr Gly Pro Cys Lys Thr Cys Thr Thr

CCT GCT CAA GGC AAC TCT ATG TTT CCC TCA TGT TGC TGT ACA   1121
Pro Ala Gln Gly Asn Ser Met Phe Pro Ser Cys Cys Cys Thr

AAA CCT ACG GAT GGA AAT TGC ACC TGT ATT CCC ATC CCA TCG   1163
Lys Pro Thr Asp Gly Asn Cys Thr Cys Ile Pro Ile Pro Ser

TCC TGG GCT TTC GCA AAA TAC CTA TGG GAG TGG GCC TCA GTC   1205
Ser Trp Ala Phe Ala Lys Tyr Leu Trp Glu Trp Ala Ser Val
```

```
CGT TTC TCT TGG CTC AGT TTA CTA GTG CCA TTT GTT CAG TGG   1247
Arg Phe Ser Trp Leu Ser Leu Leu Val Pro Phe Val Gln Trp
TTC GTA GGG CTT TCC CCC ACT GTT TGG CTT TCA GCT ATA TGG   1289
Phe Val Gly Leu Ser Pro Thr Val Trp Leu Ser Ala Ile Trp

ATG ATG TGG TAT TGG GGG CCA AGT CTG TAC AGC ATC GTG AGT   1331
Met Met Trp Tyr Trp Gly Pro Ser Leu Tyr Ser Ile Val Ser

CCC TTT ATA CCG CTG TTA CCA ATT TTC TTT TGT CTC TGG GTA   1373
Pro Phe Ile Pro Leu Leu Pro Ile Ile Phe Cys Leu Trp Val

TAC ATT TAA                                               1382
Tyr Ile
```